# EUROPÄISCHE PATENTSCHRIFT

(11) **EP 1 556 324 B1**
(45) Veröffentlichungstag und Bekanntmachung des Hinweises auf die Patenterteilung: **03.10.2007**
(21) Anmeldenummer: 03788946.6
(22) Anmeldetag: 09.10.2003
(51) Int. Cl.: C07C 49/697, C07C 49/747, A01N 35/06

(54) **2-PHENYL-2-SUBSTITUIRTE-1, 3-DIKETONE ALS SCHÄDLINGSBEKÄMPFUNGSMITTEL**
2-PHENYL-2-SUBSTITUTED-1,3-DIKETONES
1,3-DICETONES SUBSTITUES PAR 2-PHENYLE EN POSITION 2

(30) Priorität: 22.10.2002 DE 10249055
(43) Veröffentlichungstag der Anmeldung: 27.07.2005
(73) Patentinhaber: Bayer CropScience AG, 40789 Monheim (DE)
(72) Erfinder: FISCHER, Reiner, 40789 Monheim (DE); HILLEBRAND, Stefan, 41462 Neuss (DE); TRAUTWEIN, Axel, 51467 Bergisch Gladbach (DE); ULLMANN, Astrid, 51377 Leverkusen (DE); DREWES, Mark, Wilhelm, 40764 Langenfeld (DE); FEUCHT, Dieter, 40789 Monheim (DE); KONZE, Jörg, 51147 Köln (DE); KUCK, Karl-Heinz, 40764 Langenfeld (DE); WACHENDORFF-NEUMANN, Ulrike, 56566 Neuwied (DE)
(86) Internationale Anmeldenummer: PCT/EP2003/011148
(87) Internationale Veröffentlichungsnummer: WO 2004/037749

(56) Entgegenhaltungen:
- WO-A-98/39281
- WO-A-99/55673
- THOMPSON D. J.: "Reaction of tricarbonylcyclohexadieneiron complexes with cupric chloride" JOURNAL OF ORGANOMETALLIC CHEMISTRY., Bd. 108, Nr. 3, 1976, Seiten 381-383, XP002275708 CHELSEVIER-SEQUOIA S.A. LAUSANNE. in der Anmeldung erwähnt
- KAPADIA H. D. ET AL.: "Base-induced novel ring-opening of 2-alkyl-2-chloro-1,3- cyclohexanedione" INDIAN JOURNAL OF CHEMISTRY, SECTION B: ORGANIC, INCL. MEDICINAL., Bd. 20, 1981, Seiten 993-994, XP0008029388 INPUBLICATIONS & INFORMATIONS DIRECTORATE, NEW DELHI. in der Anmeldung erwähnt

## Beschreibung

Die Erfindung betrifft neue phenylsubstituierte Cyclopentan- und Cyclohexan-1,3-dion-Derivate, Verfahren und Zwischenprodukte zu ihrer Herstellung und ihre Verwendung als Schädlingsbekämpfungsmittel, Mikrobizide und/oder Herbizide.

Bekannt geworden sind cyclische in 2-Positionen halogenierte und 2-phenylsubstituierte 1,3-Diketone aus Thompsen, D.J.; J. Organomet. Chem. 108, 381-383, (1976), Kapaclia, H.D., Sunthankar, S.V.; Indian J. Chem., Sect. B, 20 B, 993 (1981). Eine Verwendung als Schädlingsbekämpfungsmittel, Mikrobizide und/oder Herbizide wurde nicht beschrieben.

Es wurden nun neue Verbindungen der Formel (I) gefunden, 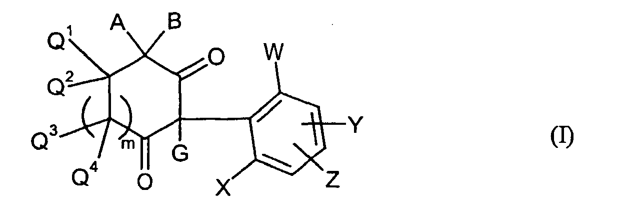 in welcher
- W: für Cyano, Halogen, Alkyl, Alkenyl, Alkinyl, Alkoxy, Halogenalkyl oder Halogenalkoxy steht,
- X: für Wasserstoff, Halogen, Alkyl, Alkoxy, Halogenalkyl, Halogenalkoxy oder Cyano steht,
- Y: für Wasserstoff, Halogen, Alkyl, Alkoxy, Halogenalkyl, Halogenalkoxy, Cyano oder gegebenenfalls substituiertes Phenyl steht,
- Z: für Wasserstoff, Halogen, Alkyl, Alkoxy, Halogenalkyl, Halogenalkoxy oder Cyano steht,
- G: für Halogen oder Nitro steht,
- m: für die Zahlen 0 oder 1 steht,
- A: für Wasserstoff, jeweils gegebenenfalls durch Halogen substituiertes Alkyl, Alkenyl, Alkoxyalkyl, Polyalkoxyalkyl, Alkylthioalkyl, gesättigtes oder ungesättigtes, gegebenenfalls substituiertes Cycloalkyl, in welchem gegebenenfalls mindestens ein Ringatom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
- B: für Wasserstoff oder Alkyl steht,
- A und B: gemeinsam mit dem Kohlenstoffatom an das sie gebunden sind, für einen gesättigten oder ungesättigten, gegebenenfalls mindestens ein Heteroatom enthaltenden unsubstituierten oder substituierten Cyclus stehen,
- A und Q¹: gemeinsam für gegebenenfalls substituiertes Alkandiyl stehen, in welchem zwei nicht direkt benachbarte Kohlenstoffatome gegebenenfalls einen weiteren gegebenenfalls substituierten Cyclus bilden,
- Q¹: für Wasserstoff, Alkyl, Alkoxyalkyl, gegebenenfalls substituiertes Cycloalkyl (worin gegebenenfalls eine Methylengruppe durch Sauerstoff oder Schwefel ersetzt ist) oder jeweils gegebenenfalls substituiertes Phenyl Hetaryl, Phenylalkyl oder Hetarylalkyl steht,
- Q², Q³, Q⁴: unabhängig voneinander für Wasserstoff oder Alkyl steht,
- Q¹ und Q²: gemeinsam mit dem Kohlenstoffatom, an das sie gebunden sind, für einen gesättigten oder ungesättigten gegebenenfalls ein Heteroatom enthaltenden unsubstituierten oder substituierten Cyclus stehen.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Die Verbindungen der Formel (I) können sowohl als Gemische als auch in Form ihrer reinen Isomeren vorliegen. Gemische der Verbindungen der Formel (I) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung von m für die Zahlen 0 und 1 ergeben sich folgende hauptsächliche Strukturen (I-1) und (I-2) 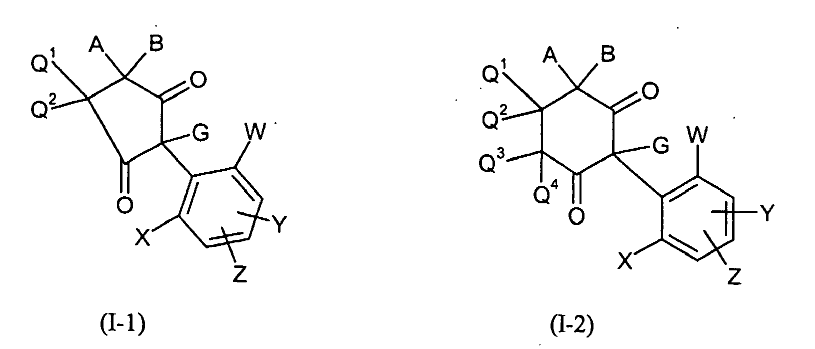 in welcher
A, B, G, Q¹, Q², Q³, Q⁴, W, X, Y und Z die oben angegebenen Bedeutungen haben.
   A) Weiterhin wurde gefunden,
      dass man Verbindungen der Formel (I-1) bis (I-2) erhält, 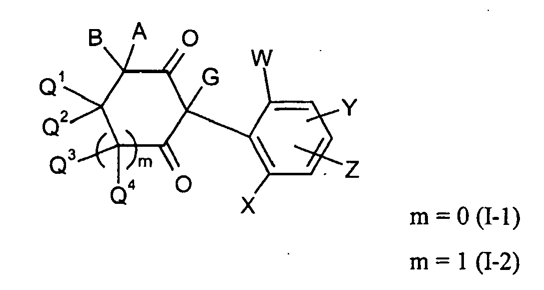 in welcher
      A, B, Q¹, Q², Q³, Q⁴, W, X, Y und Z die oben angegebene Bedeutung haben
      und
      - G: für Halogen, bevorzugt für Chlor oder Brom steht,
      wenn man Verbindungen der Formeln (II-1) bis (II-2) 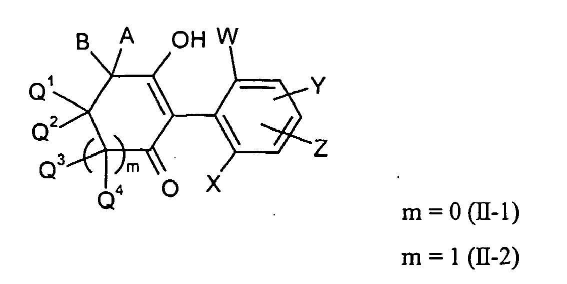 in welcher
      A, B, Q¹, Q², Q³, Q⁴, W, X, Y und Z die oben angegebene Bedeutung haben
      mit Halogenierungsmitteln in Gegenwart eines Lösungsmittels und gegebenenfalls in Gegenwart eines Radikalstarters umsetzt.
   B) Weiterhin erhält man Verbindungen der Formel (I-1) bis (1-2), 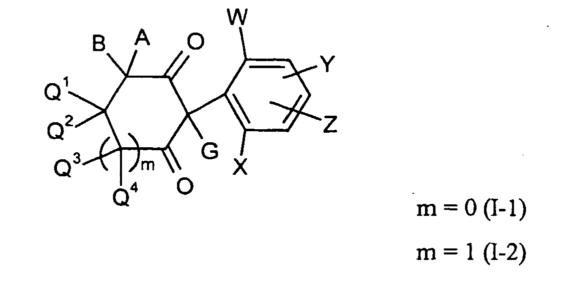
   in welcher
   A, B, Q¹, Q², Q³, Q⁴, W, X, Y und Z die oben angegebene Bedeutung haben
   und
   - G: für Nitro steht
   wenn man Verbindungen der Formeln (II-1) bis (II-2) 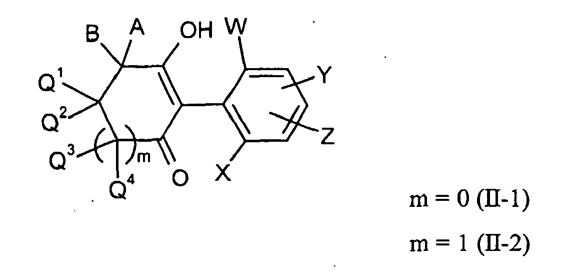 in welcher
   A, B, Q¹, Q², Q³, Q⁴, W, X, Y und Z die oben angegebene Bedeutung haben,
   mit Nitrierungsreagenzien wie z.B. rauchende Salpetersäure in Gegenwart eines Lösungsmittels umsetzt.

Die für die Verfahren A und B benötigten Verbindungen der Formeln II-1 bis II-2 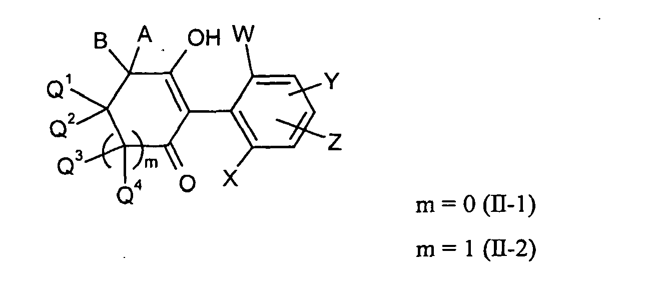 in welcher
A, B, Q¹, Q², Q³, Q⁴, W, X, Y und Z die oben angegebene Bedeutung haben,
sind teilweise bekannt: EP-A-952 149, US-4 283 348; 4 338 122; 4 436 666; 4 526 723; 4 551 547; 4 632 698; WO 96/01 798; WO 96/03 366, WO 97/14 667; WO 98/39281, WO 99/43649, WO 99/48869, WO 99/55673, US-4 175 135, 4 209 432, 4 256 657, 4 256 658, 4 256 659, 4 257 858, 4 303 669, 4 351 666, 4 409 153, 4 613 617, 4 659 372, DE-A 2 813 341, Wheeler, T.N., J. Org. Chem. 44, 4906 (1979), WO 01/09092 und WO 01/74 770 oder lassen sich nach den dort beschriebenen Verfahren synthetisieren.

Als Halogenierungsmittel kommen für das Verfahren A beispielsweise Sulfurylchlorid, Sulfurylbromid, Thionylchlorid, Thionylbromid, Imide, wie z.B. N-Bromsuccinimid oder N-Chlorsuccinimid, Chlorsulfonsäure, aber auch Hypochlorite, wie z.B. tert.-Butylhypochlorit in Frage.

Als Nitrierungsreagenzien kommen für das Verfahren B rauchende Salpetersäure, aber auch "Nitriersäuremischungen" in Frage.

Weiterhin wurde gefunden, dass die neuen Verbindungen der Formel (I) eine sehr gute Wirksamkeit als Schädlingsbekämpfungsmittel, vorzugsweise als Insektizide, Akarizide und/oder Fungizide und/oder Herbizide aufweisen und darüber hinaus gut pflanzenverträglich, insbesondere gegenüber Kulturpflanzen, sind.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- W: steht bevorzugt für Halogen, C₁-C₆-Akyl, C₂-C₆-Alkenyl, C₂-C₆-Alkinyl, C₁-C₆-Alkoxy, C₁-C₄-Halogenalkyl, C₁-C₄-Halogenalkoxy oder Cyano,
- X: steht bevorzugt für Wasserstoff, Halogen, C₁-C₆-Alkyl, C₁-C₆-Alkoxy, C₁-C₄-Halogenalkyl, C₁-C₄-Halogenalkoxy oder Cyano,
- Y: steht bevorzugt für Wasserstoff, Halogen, C₁-C₆-Akyl, C₁-C₆-Alkoxy, C₁-C₄-Halogenalkyl, C₁-C₄-Halogenalkoxy, Cyano oder für die Gruppe 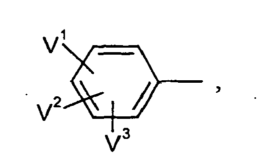
- V¹: steht bevorzugt für Wasserstoff, Halogen, C₁-C₁₂-Alkyl, C₁-C₆-Alkoxy, C₁-C₆-Alkylthio, C₁-C₆-Alkylsulfinyl, C₁-C₆-Alkylsulfonyl, C₁-C₄-Halogen-alkyl, C₁-C₄-Halogenalkoxy, Nitro, Cyano oder jeweils gegebenenfalls einfach oder mehrfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio,
- V² und V³: stehen bevorzugt unabhängig voneinander für Wasserstoff, Halogen, C₁-C₆-Alkyl, C₁-C₆-Alkoxy, C₁-C₄-Halogenalkyl oder C₁-C₄-Halogenalkoxy,
- V¹ und V²: stehen zusammen gemeinsam mit den Kohlenstoffatomen, an die sie gebunden sind bevorzugt für einen gegebenenfalls durch C₁-C₄-Alkyl oder Halogen substituierten 5- oder 6-gliedrigen Cyclus, in welchem gegebenenfalls ein bis zwei Kohlenstoffatome durch Sauerstoff, Schwefel oder Stickstoff ersetzt sein können,
- Z: steht bevorzugt für Wasserstoff, Halogen, C₁-C₆-Alkyl, C₁-C₆-Alkoxy, C₁-C₄-Halogenalkyl, C₁-C₄-Halogenalkoxy oder Cyano,
- G: steht bevorzugt für Halogen oder Nitro,
- m: steht bevorzugt für die Zahlen 0 oder 1,
- A: steht bevorzugt für Wasserstoff oder jeweils gegebenenfalls durch Halogen substituiertes C₁-C₁₂-Alkyl, C₃-C₈-Alkenyl, C₁-C₆-Alkoxy-C₁-C₄-alkyl, jeweils gegebenenfalls durch Halogen, C₁-C₄-Alkyl oder C₁-C₄-Alkoxy substituiertes C₃-C₈-Cycloalkyl oder C₃-C₆-Cycloalkyl-C₁-C₄-alkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Benzyl, Hetaryl mit 5 bis 6 Ringatomen (beispielsweise Furanyl, Pyridyl, Imidazolyl, Triazolyl, Pyrazolyl, Pyrimidyl, Thiazolyl oder Thienyl) oder Hetaryl-C₁-C₄-alkyl mit 5 bis 6 Ringatomen (beispielsweise Pyridyl, Pyrimidyl oder Thiazolyl),
- B: steht bevorzugt für Wasserstoff oder C₁-C₆-Alkyl,
- A, B: und das Kohlenstoffatom an das sie gebunden sind, stehen bevorzugt für gesättigtes C₃-C₁₀-Cycloalkyl oder ungesättigtes C₅-C₁₀-Cycloalkyl, worin gegebenenfalls ein Ringglied durch Sauerstoff oder Schwefel ersetzt ist und welche gegebenenfalls einfach oder zweifach durch C₁-C₆-Alkyl, C₃-C₈-Cycloalkyl, C₁-C₆-Halogenalkyl, C₁-C₆-Alkoxy, C₁-C₆-Alkylthio, Halogen oder Phenyl substituiert sind,
- A und Q¹: stehen gemeinsam bevorzugt für gegebenenfalls einfach oder zweifach, gleich oder verschieden durch C₁-C₄-Alkyl, C₁-C₄-Alkoxy substituiertes C₃-C₆-Alkandiyl,
- Q¹: steht bevorzugt für Wasserstoff, C₁-C₆-Alkyl, C₁-C₆-Alkoxy-C₁-C₂-alkyl, gegebenenfalls durch Fluor, Chlor, C₁-C₄-Akyl, C₁-C₂-Halogenalkyl oder C₁-C₄-Alkoxy substituiertes C₃-C₈-Cycloalkyl, worin gegebenenfalls eine Methylengruppe durch Sauerstoff oder Schwefel ersetzt ist oder jeweils gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenyl, Pyridyl, Thienyl, Thiazolyl, Phenyl-C₁-C₄-alkyl, Pyridyl-C₁-C₂-alkyl oder Thiazolyl-C₁-C₂-alkyl,
- Q², Q³, Q⁴: stehen bevorzugt unabhängig voneinander für Wasserstoff oder C₁-C₄-Alkyl,
- Q¹ und Q²: stehen bevorzugt gemeinsam mit dem Kohlenstoffatom, an das sie gebunden sind, für gegebenenfalls durch C₁-C₆-Alkyl, C₁-C₆-Alkoxy oder C₁-C₂-Halogenalkyl substituiertes C₃-C₇-Cycloalkyl, in welchem gegebenenfalls ein Ringglied durch Sauerstoff oder Schwefel ersetzt ist.

In den als bevorzugt genannten Restedefinitionen steht Halogen, auch als Substituent, wie z.B. in Halogenalkyl, für Fluor, Chlor, Brom und Iod, insbesondere für Fluor und Chlor.
- W: steht besonders bevorzugt für Fluor, Chlor, Brom, C₁-C₄-Alkyl, C₁-C₄-Alkoxy, C₁-C₂-Halogenalkyl, C₁-C₂-Halogenalkoxy oder Cyano,
- X: steht besonders bevorzugt für Wasserstoff, Fluor, Chlor, Brom, C₁-C₄-Alkyl oder C₁-C₄-Alkoxy,
- Y: steht besonders bevorzugt für Wasserstoff, Fluor, Chlor, Brom, C₁-C₆-Alkyl, C₁-C₄-Alkoxy, C₁-C₂-Halogenalkyl, C₁-C₂-Halogenalkoxy, Cyano oder für die Gruppe 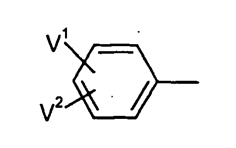
- V¹: steht besonders bevorzugt für Wasserstoff, Fluor, Chlor, Brom, C₁-C₆-Alkyl, C₁-C₄-Alkoxy, C₁-C₂-Halogenallcyl, C₁-C₂-Halogenalkoxy, Nitro, Cyano oder jeweils gegebenenfalls einfach oder zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl oder Phenoxy,
- V²: steht besonders bevorzugt für Wasserstoff, Fluor, Chlor, C₁-C₄-Alkyl, C₁-C₄-Alkoxy, C₁-C₂-Halogenalkyl oder C₁-C₂-Halogenalkoxy,
- V¹ und V²: stehen zusammen gemeinsam mit den Kohlenstoffatomen, an die sie gebunden sind, besonders bevorzugt für einen gegebenenfalls durch Fluor oder Methyl substituierten 5- oder 6-gliedrigen Cyclus, in welchem gegebenenfalls ein bis zwei Kohlenstoffatome durch Sauerstoff ersetzt sein können,
- Z: steht besonders bevorzugt für Wasserstoff, Fluor, Chlor, Brom, C₁-C₄-Alkyl, C₁-C₄-Alkoxy, C₁-C₂-Halogenalkyl, C₁-C₂-Halogenalkoxy oder Cyano,
- G: steht besonders bevorzugt für Chlor, Brom oder Nitro,
- m: steht besonders bevorzugt für die Zahlen 0 oder 1,
- A: steht besonders bevorzugt für Wasserstoff, für jeweils gegebenenfalls durch Fluor substituiertes C₁-C₈-Alkyl oder C₁-C₄-Alkoxy-C₁-C₂-alkyl, für jeweils gegebenenfalls durch Fluor, Chlor, Methyl, Ethyl oder Methoxy substituiertes C₅-C₆-Cycloalkyl oder C₃-C₆-Cycloalkyl-C₁-C₂-alkyl, in welchem gegebenenfalls ein Ringglied durch Sauerstoff oder Schwefel ersetzt ist oder jeweils gegebenenfalls durch Fluor, Chlor, Brom, C₁-C₄-Alkyl, C₁-C₂-Halogenalkyl, C₁-C₄-Alkoxy oder C₁-C₂-Halogenalkoxy substituiertes Phenyl oder Benzyl,
- B: steht besonders bevorzugt für Wasserstoff oder C₁C₄-Alkyl,
- A, B: und das Kohlenstoffatom an das sie gebunden sind, stehen besonders bevorzugt für gesättigtes C₅-C₇-Cycloalkyl, worin gegebenenfalls ein Ringglied durch Sauerstoff ersetzt ist und welches gegebenenfalls einfach durch C₁-C₄-Alkyl, Trifluormethyl oder C₁-C₄-Alkoxy substituiert ist,
mit der Maßgabe, dass dann Q¹ nur für Wasserstoff steht,
- A und Q¹: stehen gemeinsam besonders bevorzugt für gegebenenfalls einfach oder zweifach durch Methyl, Ethyl, Methoxy oder Ethoxy substitutiertes C₃-C₄-Alkandiyl,
- Q¹: steht besonders bevorzugt für Wasserstoff, C₁-C₆-Akyl, C₁-C₄-Alkoxy-C₁-C₂-alkyl, oder gegebenenfalls durch Methyl oder Methoxy substituiertes C₃-C₆-Cycloakyl, worin gegebenenfalls eine Methylengruppe durch Sauerstoff ersetzt ist, oder jeweils gegebenenfalls durch Fluor, Chlor, Brom, C₁-C₄-Alkyl, C₁-C₄-Alkoxy, Trifluormethyl oder Trifluormethoxy substituiertes Phenyl oder Benzyl,
- Q², Q³, Q⁴: stehen besonders bevorzugt unabhängig voneinander für Wasserstoff, Methyl oder Ethyl,
- Q¹ und Q²: stehen besonders bevorzugt gemeinsam mit dem Kohlenstoff, an das sie gebunden sind, für gegebenenfalls durch C₁-C₄-Alkyl oder C₁-C₄-Alkoxy substituiertes gesättigtes C₅-C₆-Cyaoalkyl, in welchem gegebenenfalls ein Ringglied durch Sauerstoff ersetzt ist,
mit der Maßgabe, dass dann A nur für Wasserstoff steht.

In den als besonders bevorzugt genannten Restedefinitionen steht Halogen, auch als Substituent, wie z.B. in Halogenalkyl für Fluor und Chlor, insbesondere für Fluor.
- W: steht ganz besonders bevorzugt für Chlor, Brom, Methyl, Ethyl, Propyl, Methoxy, Ethoxy, Trifluormethyl, Difluormethoxy, Trifluormethoxy oder Cyano,
- X: steht ganz besonders bevorzugt für Wasserstoff, Chlor, Brom, Methyl, Ethyl, Propyl, Methoxy oder Ethoxy,
- Y: steht ganz besonders bevorzugt für Wasserstoff, Fluor, Chlor, Brom, Methyl, Ethyl, n-Propyl, i-Butyl, CH(CH₃)-i-Butyl, Methoxy, Ethoxy, Trifluormethyl, Trifluormethoxy, Difluormethoxy, Cyano oder für die Gruppe 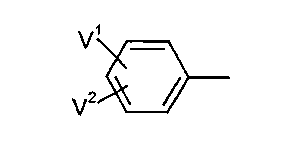
- V¹: steht ganz besonders bevorzugt für Wasserstoff, Fluor, Chlor, Brom, Methyl, Ethyl, n-Propyl, iso-Propyl, n-Butyl, iso-Butyl, tert.-Butyl, Methoxy, Ethoxy, n-Propoxy, iso-Propoxy, Trifluormethyl oder Trifluormethoxy,
- V²: steht ganz besonders bevorzugt für Wasserstoff, Fluor, Chlor, Methyl, Ethyl, n-Propyl, iso-Propyl, Methoxy, Ethoxy oder Trifluormethyl,
- Z: steht ganz besonders bevorzugt für Wasserstoff, Fluor, Chlor, Brom, Methyl, Ethyl, Propyl, Methoxy, Ethoxy, Trifluormethyl, Trifluormethoxy, Difluormethoxy oder Cyano,
- G: steht ganz besonders bevorzugt für Chlor, Brom oder Nitro (hervorgehoben für Chlor),
- m: steht ganz besonders bevorzugt für die Zahlen 0 oder 1,
- A: steht ganz besonders bevorzugt für Wasserstoff, Methyl, Ethyl, n-Propyl, iso-Propyl, n-Butyl, iso-Butyl, Methoxymethyl oder Ethoxymethyl,
- B: steht ganz besonders bevorzugt für Wasserstoff, Methyl oder Ethyl,
- A, B: und das Kohlenstoffatom, an das sie gebunden sind, stehen ganz besonders bevorzugt für gesättigtes C₅-C₇-Cyloalkyl, in welchem gegebenenfalls ein Ringglied durch Sauerstoff ersetzt ist und welches gegebenenfalls einfach durch Methyl, Ethyl, Isopropyl, Trifluormethyl, Methoxy, Ethoxy, n-Propoxy, n-Butoxy oder Isobutoxy substituiert ist,
mit der Maßgabe, dass dann Q¹, Q², Q³ und Q⁴ nur für Wasserstoff stehen,
- A und Q¹: stehen gemeinsam ganz besonders bevorzugt für C₃-C₄-Alkandiyl,
- Q¹: steht ganz besonders bevorzugt für Wasserstoff, Methyl, Ethyl, n-Propyl, iso-Propyl, n-Hexyl, Cyclopropyl, Cyclopentyl oder Cyclohexyl,
- Q², Q³, Q⁴: stehen ganz besonders bevorzugt unabhängig voneinander für Wasserstoff, Methyl oder Ethyl,
- Q¹ und Q²: stehen ganz besonders bevorzugt gemeinsam mit dem Kohlenstoff, an den sie gebunden sind, für gegebenenfalls durch Methyl, Ethyl, Methoxy, Ethoxy, n-Propoxy oder n-Butoxy substituiertes gesättigtes C₅-C₆-Cycloalkyl, in welchem gegebenenfalls ein Ringglied durch Sauerstoff ersetzt ist,
mit der Maßgabe, dass dann A, B, Q³ und Q⁴ nur für Wasserstoff stehen.
- W: steht hervorgehoben für Methyl oder Chlor,
- X: steht hervorgehoben für Wasserstoff oder Methyl,
- Y: steht hervorgehoben für gegebenenfalls einfach oder zweifach durch Chlor oder Fluor substituiertes Phenyl, für Chlor, Brom, Methyl, Ethyl, n-Propyl, i-Butyl, CH(CH₃)-i-Butyl oder Trifluormethyl,
- Z: steht hervorgehoben für Wasserstoff,
- G: steht hervorgehoben für Chlor,
- m: steht hervorgehoben für 0 oder 1,
- A: steht hervorgehoben für Wasserstoff oder Methyl,
- B: steht hervorgehoben für Wasserstoff oder Methyl,
- A, B: und das Kohlenstoffatom, an das sie gebunden sind stehen hervorgehoben für C₅-C₇-Cycloalkyl, mit der Maßgabe, dass dann Q¹ und Q² nur für Wasserstoff stehen,
- Q¹: steht hervorgehoben für Wasserstoff, Methyl, Ethyl, i-Propyl oder n-Hexyl,
- Q²: steht hervorgehoben für Wasserstoff oder Methyl,
- Q¹, Q²: und das Kohlenstoffatom, an das sie gebunden sind stehen hervorgehoben für C₅-C₆-Cycloalkyl, mit der Maßgabe, dass dann A und B nur für Wasserstoff stehen,
- Q³: steht hervorgehoben für Wasserstoff,
- Q⁴: steht hervorgehoben für Wasserstoff.

Im Fall der Verbindungen der Formel (I-2) stehen A und B insbesondere für Wasserstoff.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (1), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß hervorgehoben werden die Verbindungen der Formel (I), in welchen eine Kombination der vorstehend als hervorgehoben aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Verwendet man beispielsweise gemäß Verfahren (A) 2-(2-Methyl-5-brom)-phenyl-4,4-pentamethylen-cyclopentan-1,3-dion oder dessen Enol als Ausgangsstoff, so kann der Verlauf des erfindungsgemäßen Verfahrens durch folgendes Reaktionsschema wiedergegeben werden:

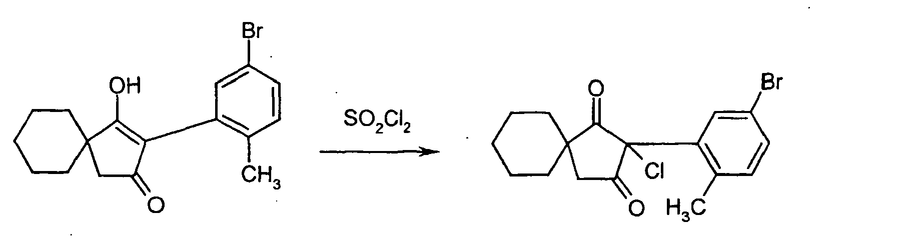

Verwendet man beispielsweise gemäß Verfahren (B) 2-[2-Methyl-5-(4-chlorphenyl)-phenyl]-4,4-pentamethylen-cyclopentan-1,3-dion oder dessen Enol, so kann der Verlauf des erfindungsgemäßen Verfahrens durch folgendes Reaktionsschema wiedergegeben werden:

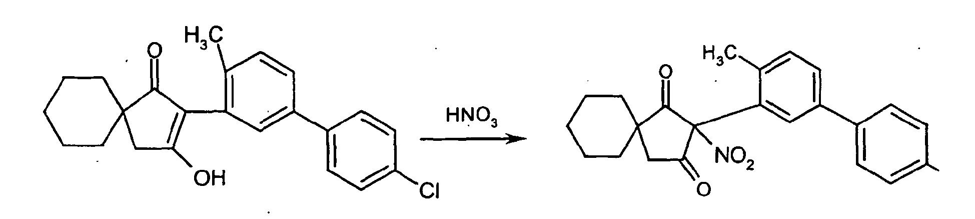

Das Verfahren (A) ist dadurch gekennzeichnet, dass man Verbindungen der Formel (II), in welcher A, B, Q¹, Q², Q³, Q⁴, W, X, Y, Z und m die oben angegebenen Bedeutungen haben, in Gegenwart eines Verdünnungsmittel und eines Halogenierungsmittels und gegebenenfalls eines Radikalstarters umsetzt. Als Radikalstarter können beispielsweise Benzoylperoxid oder Azobisisobutyronitril verwendet werden.

Als Verdünnungsmittel können bei dem erfindungsgemäßen Verfahren (A) alle inerten organischen Solventien eingesetzt werden. Vorzugsweise verwendbar sind Kohlenwasserstoffe wie Benzol, Toluol und Xylol, ferner Ether, wie Dibutylether, Tetrahydrofuran, Dioxan, Glykoldimethylether und Diglykoldimethylether, außerdem halogenierte Kohlenwasserstoffe, wie Dichlormethan, Chloroform, Tetrachlorkohlenstoff, Dichlorethan, Chlorbenzol, Dichlorbenzol, aber auch Ester wie Ethylacetat.

Als Halogenierungsmittel kommen für das Verfahren A beispielsweise Sulfurylchlorid, Sulfurylbromid, Thionylchlörid, Thionylbromid, Imide wie z.B. N-Bromsuccinimid, N-Chlorsuccinimid, weiterhin Chlorsulfonsäure, aber auch Hypochlorite wie z.B. tert.-Butylhypochlorit in Frage.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen -40°C und 150°C, vorzugsweise zwischen 0°C und 100°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formel (II) und die Halogenierungsmittel im allgemeinen in etwa äquimolaren Mengen ein. Es ist jedoch auch möglich, die eine oder andere Komponente in einem größeren Überschuss (bis zu 3 Mol) zu verwenden.

Die Reinigung erfolgt in der Regel nach wässriger Aufarbeitung, durch Kristallisation oder durch chromatographische Reinigung an Kieselgel.

Das Verfahren (B) ist dadurch gekennzeichnet, dass Verbindungen der Formel (II), in welcher A, B, Q¹, Q², Q³, Q⁴, W, X, Y, Z und m die oben angegebenen Bedeutungen haben, in Gegenwart eines Verdünnungsmittels und in Gegenwart eines Nitrierungsmittels umsetzt.

Als Verdünnungsmittel können bei dem erfindungsgemäßen Verfahren (B) alle inerten organischen Solventien eingesetzt werden. Vorzugsweise verwendbar sind halogenierte Kohlenwasserstoffe wie Methylenchlorid, Chloroform, Dichlorbenzol, Dichlorethan.

Als Nitrierungsmittel kommen Nitriersäuren bevorzugt rauchende Salpetersäure in Frage.

Die Reaktionstemperaturen können bei der Durchführung des erfindungsgemäßen Verfahrens (B) innerhalb eines größeren Bereiches variiert werden. Im allgemeinen arbeitet man bei Temperaturen zwischen -50°C und 150°C, vorzugsweise zwischen 0°C und 80°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der Formel (II) und das Nitrierungsreagenz im allgemeinen in etwa äquimolaren Mengen ein. Es ist jedoch auch möglich, die eine oder andere Komponenten in einem größeren Überschuss (bis zu 5 Mol) zu verwenden.

Die Reinigung erfolgt nach üblichen Aufarbeitung durch Kristallisation oder chromatografische Reinigung an Kieselgel.

Die Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren und Nematoden, die in der Landwirtschaft, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spp..

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Acheta domesticus, Gryllotalpa spp., Locusta migratoria migratorioides, Melanoplus spp., Schistocerca gregaria.

Aus der Ordnung der Blattaria z.B. Blatta orientalis, Periplaneta americana, Leucophaea maderae, Blattella germanica.

Aus der Ordnung der Dermaptera z.B. Forficula auricularia.

Aus der Ordnung der Isoptera z.B. Reticulitermes spp..

Aus der Ordnung der Phthiraptera z.B. Pediculus humanus corporis, Haematopinus spp., Linognathus spp., Trichodectes spp., Damalinia spp..

Aus der Ordnung der Thysanoptera z.B. Hercinothrips femoralis, Thrips tabaci, Thrips palmi, Frankliniella accidentalis.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..

Aus der Ordnung der Siphonaptera z.B. Xenopsylla cheopis, Ceratophyllus spp..

Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die erfindunsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injezieren und bei Vermehrungsmaterial, insbesondere bei Samen, weiterhin durch ein- oder mehrschichtiges Umhüllen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
**Fungizide:**
   Aldimorph, Ampropylfos, Ampropylfos-Kalium, Andoprim, Anilazin, Azaconazol, Azoxystrobin,
   Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
   Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
   Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
   Ediphenphos, Epoxiconazol, Etaconazol, Ethirimol, Etridiazol,
   Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
   Guazatin,
   Hexachlorobenzol, Hexaconazol, Hymexazol,
   Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
   Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrothal-isopropyl, Nuarimol,
   Ofurace, Oxadixyl, Oxamocarb, Oxolinicacid, Oxycarboxim, Oxyfenthiin,
   Paclobutrazol, Pefurazoat, Penconazol, Pencycuron, Phosdiphen, Picoxystrobin, Pimaricin, Piperalin, Polyoxin, Polyoxorim, Probenazol, Prochloraz, Procymidon, Propamocarb, Propanosine-Natrium, Propiconazol, Propineb, Pyraclostrobin, Pyrazophos, Pyrifenox, Pyrimethanil, Pyroquilon, Pyroxyfur,
   Quinconazol, Quintozen (PCNB),
   Schwefel und Schwefel-Zubereitungen,
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Trifloxystrobin, Triflumizol, Triforin, Triticonazol,
   Uniconazol,
   Validamycin A, Vinclozolin, Viniconazol,
   Zarilamid, Zineb, Ziram sowie
   Dagger G,
   OK-8705,
   OK-8801,

   α-(1,1-Dimethylethyl)-β-(2-phenoxyethyl)-1H-1,2,4-triazol-1-ethanol,
   α-(2,4-Dichlorphenyl)-β-fluor-b-propyl-1H-1,2,4-triazol-1-ethanol,
   α-(2,4-Dichlorphenyl)-β-methoxy-a-methyl-1H-1,2,4-triazol-1-ethanol,
   α-(5-Methyl-1,3-dioxan-5-yl)-β-[[4-(trifluormethyl)-phenyl]-methylen]-1H-1,2,4-triazol-1-ethanol,

   (5RS,6RS)-6-Hydroxy-2,2,7,7-tetramethyl-5-(1H-1,2,4-triazol-1-yl)-3-octanon,
   (E)-a-(Methoxyimino)-N-methyl-2-phenoxy-phenylacetamid,

   {2-Methyl-1-[[[1-(4-methylphenyl)-ethyl]-amino]-carbonyl]-propyl}-carbaminsäure-1-isopropylester

   1-(2,4-Dichlorphenyl)-2-(1H-1,2,4-triazol-1-yl)-ethanon-O-(phenylmethyl)-oxim,
   1-(2-Methyl-1-naphthalenyl)-1H-pyrrol-2,5-dion,
   1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlorphenyl)-1,3-dioxolan-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)-methoxy]-phenyl]-ethenyl]-1H-imidazol,
   1-Methyl-5-nonyl-2-(phenylmethyl)-3-pyrrolidinol,
   2',6'-Dibrom-2-methyl-4'-trifluormethoxy-4'-trifluor-methyl-1,3-thiazol-5-carboxanilid,
   2,2-Dichlor-N-[1-(4-chlorphenyl)-ethyl]-1-ethyl-3-methyl-cyclopropancarboxamid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlormethyl)-1,3,4-thiadiazol,
   2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
   2-Aminobutan,
   2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid,
   2-Phenylphenol(OPP),
   3,4-Dichlor-1-[4-(difluormethoxy)-phenyl]-1H-pyrrol-2,5-dion,
   3,5-Dichlor-N-[cyan[(1-methyl-2-propynyl)-oxy]-methyl]-benzamid,
   3-(1,1-Dimethylpropyl-1-oxo-1H-inden-2-carbonitril,
   3-[2-(4-Chlorphenyl)-5-ethoxy-3-isoxazolidinyl]-pyridin,
   4-Chlor-2-cyan-N,N-dimethyl-5-(4-methylphenyl)-1H-imidazol-1-sulfonamid,
   4-Methyl-tetrazolo[1,5-a]quinazolin-5(4H)-on,
   8-(1,1-Dimethylethyl)-N-ethyl-N-propyl-1,4-dioxaspiro[4.5]decan-2-methanamin,
   8-Hydroxychinolinsulfat,
   9H-Xanthen-9-carbonsäure-2-[(phenylamino)-carbonyl]-hydrazid,
   bis-(1-Methylethyl)-3-methyl-4-[(3-methylbenzoyl)-oxy]-2,5-thiophendicarboxylat,
   cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholin-hydrochlorid,
   Ethyl-[(4-chlorphenyl)-azo]-cyanoacetat,
   Kaliumhydrogencarbonat,
   Methantetrathiol-Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,3-Dichlor-4-hydroxyphenyl)-1-methyl-cyclohexancarboxamid.
   N-(2,6-Dimethylphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2- Trichlor-1-[(chloracetyl)-amino]-ethyl]-benzamid,
   N-[3-Chlor-4,5-bis-(2-propinyloxy)-phenyl]-N'-methoxy-methanimidamid,
   N-Formyl-N-hydroxy-DL-alanin -Natriumsalz,
   O,O-Diethyl-[2-(dipropylamino)-2-oxoethyl]-ethylphosphoramidothioat,
   O-Methyl-S-phenyl-phenylpropylphosphoramidothioat,
   S-Methyl-1,2,3-benzothiadiazol-7-carbothioat,
   spiro[2H]-1-Benzopyran-2,1'(3'H)-isobenzofuran]-3'-on,
   4-[3,4-Dimethoxyphenyl)-3-(4-fluorphenyl)-acryloyl]-morpholin
**Bakterizide:**
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Acephate, Acetamiprid, Acequinocyl, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
   Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, Bistrifluron, BPMC, Bromophos A, Bufencarb, Buprofezin, Butathiofos, Butocarboxim, Butylpyridaben,
   Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Chromafenozide, Cis-Resmethrin, Cispermethrin, Clocythrin, Cloethocarb, Clofentezine, Clothianidine, Cyanophos, Cycloprene, Cycloprothrin, Cyfluthrin, Cyhalothrin, Cyhexatin, Cypermethrin, Cyromazine,
   Deltamethrin, Demeton M, Demeton S, Demeton-S-methyl, Diafenthiuron, Diazinon, Dichlorvos, Dicofol, Diflubenzuron, Dimethoat, Dimethylvinphos, Dinetofuran, Diofenolan, Disulfoton, Docusat-sodium, Dofenapyn,
   Eflusilanate, Emamectin, Empenthrin, Endosulfan, Entomopfthora spp., Esfenvalerate, Ethiofencarb, Ethion, Ethiprole, Ethoprophos, Etofenprox, Etoxazole, Etrimfos,
   Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenthion" Fenvalerate, Fipronil, Fluazinam, Fluazuron, Flubrocythrinate, Flucycloxuron, Flucythrinate, Flufenoxuron, Flumethrin, Flupyrazofos, Flutenzine, Fluvalinate, Fonophos, Fosmethilan, Fosthiazate, Fubfenprox, Furathiocarb,
   Granuloseviren
   Halofenozide, HCH, Heptenophos, Hexaflumuron, Hexythiazox, Hydroprene,
   Imidacloprid, Indoxacarb, Isazofos, Isofenphos, Isoxathion, Ivermectin,
   Kernpolyederviren
   Lambda-cyhalothrin, Lufenuron
   Malathion, Mecarbam, Metaldehyd, Methamidophos, Metharhizium anisopliae, Metharhizium flavoviride, Methidathion, Methiocarb, Methoprene, Methomyl, Methoxyfenozide, Metolcarb, Metoxadiazone, Mevinphos, Milbemectin, Milbemycin, Monocrotophos,
   Naled, Nitenpyram, Nithiazine, Novaluron
   Omethoat, Oxamyl, Oxydemethon M
   Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen,
   Quinalphos,
   Ribavirin
   Salithion, Sebufos, Silafluofen, Spinosad, Spirodiclofen, Sulfotep, Sulprofos,
   Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon Theta-cypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
   Vamidothion, Vaniliprole, Verticillium lecanii

   YI 5302
   Zeta-cypermethrin, Zolaprofos

   (1R-cis)-[5-(Phenylmethyl)-3-furanyl]-methyl-3-[(dihydro-2-oxo-3(2H)-furanyliden)-methyl]-2,2-dimethylcyclopropancarboxylat
   (3-Phenoxyphenyl)-methyl-2,2,3,3-tetramethylcyclopropanecarboxylat
   1-[(2-Chlor-5-thiazolyl)methyl]tetrahydro-3,5-dimethyl-N-nitro-1,3,5-triazin-2(1H)-imin

   2-(2-Chlor-6-fluorphenyl)-4-[4-(1,1-dimethylethyl)phenyl]-4,5-dihydro-oxazol
   2-(Acetlyoxy)-3-dodecyl-1,4-naphthalindion
   2-Chlor-N-[[[4-(1-phenylethoxy)-phenyl]-amino]-carbonyl]-benzamid
   2-Chlor-N-[[[4-(2,2-dichlor-1,1-difluorethoxy)-phenyl]-amino]-carbonyl]-benzamid
   3-Methylphenyl-propylcarbamat
   4-[4-(4-Ethoxyphenyl)-4-methylpentyl]-1-fluor-2-phenoxy-benzol
   4-Chlor-2-(1,1-dimethylethyl)-5-[[2-(2,6-dimethyl-4-phenoxyphenoxy)ethyl]thio]-3(2H)-pyridazinon
   4-Chlor-2-(2-chlor-2-methylpropyl)-5-[(6-iod-3-pyridinyl)methoxy]-3(2H)-pyridazinon
   4-Chlor-5-[(6-chlor-3-pyridinyl)methoxy]-2-(3,4-dichlorphenyl)-3(2H)-pyridazinon
   Bacillus thuringiensis strain EG-2348
   Benzoesäure [2-benzoyl-1-(1,1-dimethylethyl)-hydrazid
   Butansäure 2,2-dimethyl-3-(2,4-dichlorphenyl)-2-oxo-1-oxaspiro[4.5]dec-3-en-4-yl-ester
   [3-[(6-Chlor-3-pyridinyl)methyl]-2-thiazolidinyliden]-cyanamid
   Dihydro-2-(nitromethylen)-2H-1,3-thiazine-3(4H)-carboxaldehyd
   Ethyl-[2-[[1,6-dihydro-6-oxo-1-(phenylmethyl)-4-pyridazinyl]oxy]ethyl]-carbamat
   N-(3,4,4-Trifluor-1-oxo-3-butenyl)-glycin
   N-(4-Chlorphenyl)-3-[4-(difluormethoxy)phenyl]-4,5-dihydro-4-phenyl-1H-pyrazol-1-carboxamid
   N-[(2-Chlor-5-thiazolyl)methyl]-N'-methyl-N"-nitro-guanidin
   N-Methyl-N'-(1-methyl-2-propenyl)-1,2-hydrazindicarbothioamid
   N-Methyl-N'-2-propenyl-1,2-hydrazindicarbothioamid
   O,O-Diethyl-[2-(dipropylamino)-2-oxoethyl]-ethylphosphoramidothioat
   N-Cyanomethyl-4-trifluormethyl-nicotinamid
   3,5-Dichlor-1-(3,3-dichlor-2-propenyloxy)-4-[3-(5-trifluormethylpyridin-2-yloxy)-propoxy]-benzol

Auch eine Mischung mit anderen bekannten Wirkstoffen, wie Herbiziden oder mit Düngemitteln und Wachstumsregulatoren Safenern bzw. Semichemicals ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen).

Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp.; Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari, (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Stemostoma spp., Varroa spp..

Aus der Ordnung der Actinedida (Prostigmata) und Acaridida (Astigmata) z.B. Acarapis spp., Cheyletiella spp., Omithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
   Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus. Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophehon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid, Triflumuron, Chlothianidin, Spinosad, Tefluthrin,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Sealpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflußkrebse) zusammengefaßt werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis-(trialkylzinn)-sulfiden, Tri-*n*-butylzinnlaurat, Tri-*n*-butylzinnchlorid, Kupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
   2-*tert*.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
   Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie Azaconazole, Cyproconazole, Epoxyconazole, Hexaconazole, Metconazole, Propiconazole und Tebuconazole;
Molluskizide wie
   Fentinacetat, Metaldehyd, Methiocarb, Niclosamid, Thiodicarb und Trimethacarb, Fe-chelate;
   oder herkömmliche Antifouling-Wirkstoffe wie
   4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wäßrigen System, Vinylchlorid/Vinylacetat-Copolymersysteme in Form wäßriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentomä z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coloptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfem, Gel-und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten auf wachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise
Acetochlor, Acifluorfen(-sodium), Aclonifen, Alachlor, Alloxydim(-sodium), Ametryne, Amicarbazone, Amidochlor, Amidosulfuron, Anilofos, Asulam, Atrazine, Azafenidin, Azimsulfuron, Beflubutamid, Benazolin(-ethyl), Benfuresate, Bensulfuron(-methyl), Bentazon, Benzfendizone, Benzobicyclon, Benzofenap, Benzoylprop (-ethyl), Bialaphos, Bifenox, Bispyribac(-sodium), Bromobutide, Bromofenoxim, Bromoxynil, Butachlor, Butafenacil(-allyl), Butroxydim, Butylate, Cafenstrole, Caloxydim, Carbetamide, Carfentrazone(-ethyl), Chlomethoxyfen, Chloramben, Chloridazon, Chlorimuron (-ethyl), Chlomitrofen, Chlorsulfuron, Chlortoluron, Cinidon(-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn; Dimethenamid, Dimexyflam, Dinitramine, Diphenamid, Diquat, Dithiopyr, Diuron, Dymron, Epropodan, EPTC, Esprocarb, Ethalfluralin, Ethametsulfuron(-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulf uron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron,- Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Für die Mischungen kommen weiterhin bekannte Safener in Frage, beispielsweise:

AD-67, BAS-145138, Benoxacor, Cloquintocet (-mexyl), Cyometrinil, 2,4-D, DKA-24, Dichlormid, Dymron, Fenclorim, Fenchlorazol (-ethyl), Flurazole, Fluxofenim, Furilazole, Isoxadifen (-ethyl), MCPA, Mecoprop (-P), Mefenpyr (-diethyl), MG-191, Oxabetrinil, PPG-1292, R-29148.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helmninthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokolation mit unerwünschten Mikroorgansimen weitgehende Resistenz gegen diese Mirkroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäure-ester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phosphölipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
**Fungizide:**
   2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarbisopropyl; Benzamacril; Benzamacril-isobutyl; Bilanafos; Binapacryl; Biphenyl; Bitertanol; Blasticidin-S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesil; Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosinesodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-chlorophenyl)-2-propynyl]oxy]-3-methoxyphenyl]ethyl]-3-methyl- 2-[(methylsulfonyl)amino]-butanamide; 1-(1-naphthalenyl)-1H-pyrrole-2,5-dione; 2,3,5,6-tetrachloro-4-(methylsulfonyl)-pyridine; 2-amino-4-methyl-N-phenyl-5-thiazolecarboxamide; 2-chloro-N-(2,3-dihydro-1,1,3-trimethyl-1H-inden-4-yl)-3-pyridincarboxam ide; 3,4,5-trichloro-2,6-pyridinedicarbonitrile; Actinovate; cis-1-(4-chlorophenyl)-2-(1H-1,2,4-triazole-1-yl)-cyaoheptanol; methyl 1-(2,3-dihydro-2,2-dimethyl-1H-inden-1-yl)-1H-imidazole-5-carboxylate; monopotassium carbonate; N-(6-methoxy-3-pyridinyl)-cyclopropanecarboxamide; N-butyl-8-(1,1-dimethylethyl)-1-oxaspiro[4.5]decan-3-amine; Sodium tetrathiocarbonate;
   sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxine-copper.
**Bakterizide:**
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin,
   Bacillus popilliae, Bacillus .sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben,
   Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, CisPermethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine,
   DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimethoate, Dimethylvinphos, Dinobuton, Dinocap, Dinotefuran, Diofenolan, Disulfoton, Docusat-sodium, Dofenapyn, DOWCO-439,
   Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos,
   Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb,
   Gamma-HCH, Gossyplure, Grandlure, Granuloseviren,
   Halfenprox, Halofenozide, HCH, HCN-801, Heptenophos, Hexaflumuron, Hexythiazox, Hydramethylnone, Hydroprene,
   IKA-2002, Imidacloprid, Imiprothrin, Indoxacarb, Iodofenphos, Iprobenfos, Isazofos, Isofenphos, Isoprocarb, Isoxathion, Ivermectin,
   Japonilure,
   Kadethrin, Kempolyederviren, Kinoprene,
   Lambda-Cyhalothrin, Lindane, Lufenuron,
   Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metolcarb, Metoxadiazone, Mevinphos, Milbemectin, Milbemycin, MKI-245, MON-45700, Monocrotophos, Moxidectin, MTI-800,
   Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron,
   OK-5101, OK-5201, OK-9601, OK-9602, OK-9701, OK-9802, Omethoate, Oxamyl, Oxydemeton-methyl,
   Paecilomyces fumosoroseus, Parathion-methyl, Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Prallethrin, Profenofos, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen,
   Quinalphos,
   Resmethrin, RH-5849, Ribavirin, RU-12457, RU-15525,
   S-421, S-1833, Salithion, Sebufos, SI-0009, Silafluofen, Spinosad, Spirodiclofen, Spiromesifen, Sulfluramid, Sulfotep, Sulprofos, SZI-121,
   Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (1R-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Triflumuron, Trimethacarb,
   Vamidothion, Vaniliprole, Verbutin, Verticillium lecanii,
   WL-108477, WL-40027,
   YI-5201, YI-5301, YI-5302,
   XMC, Xylylcarb,
   ZA-3274, Zeta-Cypermethrin, Zolaprofos, ZXI-8901,
die Verbindung 3-Methyl-phenyl-propylcarbamat (Tsumacide Z),
die Verbindung 3-(5-Chlor-3-pyridinyl)-8-(2,2,2-trifluorethyl)-8-azabicyclo[3.2.1]-octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923),
sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sproßpilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes,

Microsporon-Spezies wie Microsporon canis und audouinii. Die Aufzählung dieser Pilze stellt keinesfalls eine Beschränkung des erfaßbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Die Herstellung und die Verwendung der erfindungsgemäßen Wirkstoffe geht aus den nachfolgenden Beispielen hervor.

### Herstellungsbeispiele

### Beispiel I-1-1

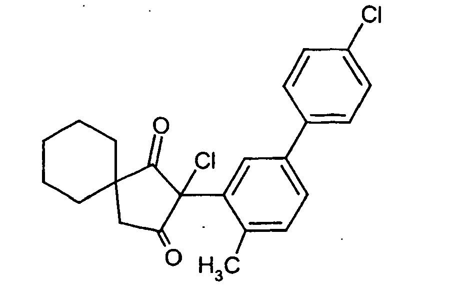

Zu 0,73 g der Verbindung gemäß Herstellungsbeispiel I-7-a-2 aus EP-A 1 066 258 in 20 ml wasserfreiem Chloroform gibt man 0,16 ml Sulfurylchlorid in 0,5 ml wasserfreiem Chloroform. Man rührt 20 Minuten.

Die Reaktionslösung wird mit Natriumhydrogencarbonatlösung gewaschen, getrocknet und anschließend wird das Lösungsmittel abdestilliert. Das Rohprodukt wird mit Cyclohexan/Essigsäureethylester verrührt und kristallisiert aus.
Ausbeute: 0,59 g (74 % der Theorie), Fp. 135-136°C

In Analogie zu Beispiel (I-1-1) und gemäß der allgemeinen Angaben zur Herstellung erhält man folgende Verbindungen der Formel (I-1) und Formel (I-2)

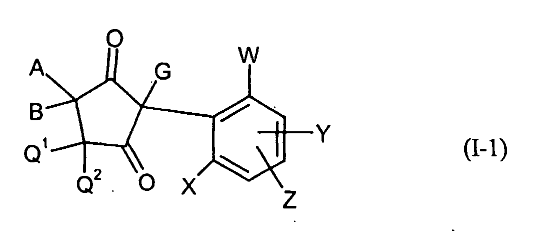

| **Bsp.-Nr.** | **W** | **X** | **Y** | **Z** | **A** | **B** | **Q¹** | **Q²** | **G** | **Fp. °C** |
|---|---|---|---|---|---|---|---|---|---|---|
| I-1-2 | CH₃ | H | 5-Br | H | - (CH₂)₅ - | | H | H | Cl | 150-151 |
| I-1-3 | CH₃ | H | 5-(4-Cl-C₆H₄) | H | - (CH₂)₄ - | | H | H | Cl | 188-189 |
| I-1-4 | CH₃ | H | 5-(4-Cl-C₆H₄) | H | - (CH₂)₆ - | | H | H | Cl | 159-161 |
| I-1-5 | CH₃ | H | 5-(4-Cl-C₆H₄) | H | CH₃ | CH₃ | H | H | Cl | 86-88 |
| I-1-6 | CH₃ | H | 5-(3,5-Cl₂-C₆H₃) | H | -(CH₂)₅- | | H | H | Cl | 161-162 |
| I-1-7 | CH₃ | H | 5-(3-Cl-C₆H₄) | H | -(CH₂)5- | | H | H | Cl | 125-126 |
| I-1-8 | Cl | CH₃ | 4-CH₃ | H | -(CH₂)₅- | | H | H | Cl | Öl |

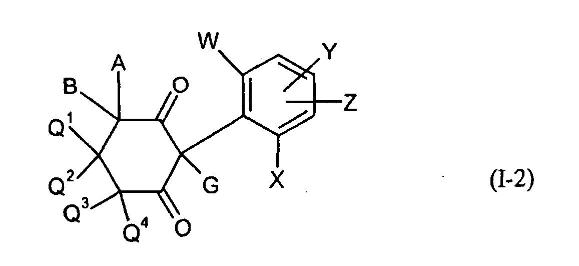

| **Bsp.-Nr.** | **W** | **X** | **Y** | **Z** | **A** | **B** | **Q¹** | **Q²** | **Q³** | **Q⁴** | **G** | **Fp. °C** |
|---|---|---|---|---|---|---|---|---|---|---|---|---|
| I-2-1 | Cl | H | 5-(4-Cl-C₆H₄) | H | H | H | CH₃ | CH₃ | H | H | Cl | 161-162 |
| I-2-2 | CH₃ | H | 5-(4-Cl-C₆H₄) | H | H | H | -(CH₂)₄- | | H | H | Cl | 144-145 |
| I-2-3 | CH₃ | H | 5-(4-Cl-C₆H₄) | H | H | H | CH₃ | CH₃ | H | H | Cl | 186-188 |
| I-2-4 | Cl | H | 5-Br | H | H | H | -(CH₂)₄- | | H | H | Cl | 141-143 |
| I-2-5 | Cl | H | 5-(4-Cl-C₆H₄) | H | H | H | -(CH₂)₄- | | H | H | Cl | 138-140 |
| I-2-6 | Cl | H | 5-Br | H | H | H | i-C₃H₇ | H | H | H | Cl | 178-179 |
| I-2-7 | Cl | H | 5-CF₃ | H | H | H | -(CH₂)₄- | | H | H | Cl | 112-116 |
| I-2-8 | CH₃ | H | 5-(4-Cl-C₆H₄) | H | H | H | C₆H₁₃ | H | H | H | Cl | 112 |
| I-2-9 | CH₃ | H | 5-C₂H₅ | H | H | H | -(CH₂)₄- | | H | H | Cl | Öl |
| I-2-10 | CH₃ | H | 5-(2,5-F₂C₆H₃) | H | H | H | -(CH₂)₄- | | H | H | Cl | 129 |
| I-2-11 | CH₃ | H | 5-i-C₄H₉ | H | H | H | -(CH₂)₄- | | H | H | Cl | Öl |
| I-2-12 | CH₃ | H | 5-i-C₄H₉ | H | H | H | C₂H₅ | CH₃ | H | H | Cl | Öl |
| I-2-13 | CH₃ | H | 5-n-C₃H₇ | H | H | H | -(CH₂)₄- | | H | H | Cl | Öl |
| I-2-14 | Cl | H | 3-Cl | H | H | H | -(CH₂)₄- | | H | H | Cl | Öl |
| I-2-15 | CH₃ | H | 5-CHCH₃i-C₄H₉ | H | H | H | -(CH₂)₄- | | H | H | Cl | Harz |
| I-2-16 | CH₃ | H | 5-(4-Cl-C₆H₄) | H | H | H | -(CH₂)₅- | | H | H | Cl | 187-188 |
| I-1-17 | CH₃ | H | 5-(3-Cl-C₆H₄) | H | H | H | CH₃ | CH₃ | H | H | Cl | 126-129 |

### Beispiel A

### Meloidogyne-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gefäße werden mit Sand, Wirkstofflösung, Meloidogyne incognita-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigen z. B. die folgenden Verbindungen der Herstellungsbeispiele gute Wirksamkeit:

**Tabelle A**

| pflanzenschädigende Nematoden | | |
|---|---|---|
| **Meloidogyne-Test** | | |
| **Wirkstoffe** | **Wirkstoffkonzentration in ppm** | **Wirkung in % nach 14^{d}** |
| Bsp. I-2-6 | 20 | 80 |
| Bsp. I-2-1 | 20 | 90 |

### Beispiel B

### Myzus-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z. B. die folgende Verbindung der Herstellungsbeispiele gute Wirksamkeit:

**Tabelle B**

| pflanzenschädigende Insekten | | |
|---|---|---|
| **Myzus-Test** | | |
| **Wirkstoffe** | **Wirkstoffkonzentration in ppm** | **Abtötungsgrad in % nach 6^{d}** |
| Bsp. I-1-1 | 500 | 80 |

### Beispiel C

### Phaedon-Larven-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven des Meerrettichblattkäfers (Phaedon cochleariae) besetzt, solange die Blätter noch feucht sind.

Nach der gewünschten Zeit wird die Abtötung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele gute Wirksamkeit:

**Tabelle C**

| pflanzenschädigende Insekten | | |
|---|---|---|
| **Phaedon-Larven-Test** | | |
| **Wirkstoffe** | **Wirkstoffkonzentration in ppm** | **Abtötungsgrad in % nach 7^{d}** |
| Bsp. I-2-5 | 500 | 100 |
| Bsp. I-1-1 | 500 | 100 |
| Bsp. I-2-2 | 500 | 100 |
| Bsp. I-2-1 | 500 | 100 |
| Bsp. I-2-3 | 500 | 100 |

### Beispiel D

### Spodoptera frugiperda-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100 %, dass alle Raupen abgetötet Wurden; 0 % bedeutet, dass keine Raupen abgetötet wurden.

Bei diesem Test zeigen z. B. die folgenden Verbindungen der Herstellungsbeispiele gute Wirksamkeit:

**Tabelle D**

| pflanzenschädigende Insekten | | |
|---|---|---|
| **Spodoptera frugiperda -Test** | | |
| **Wirkstoffe** | **Wirkstoffkonzentration in ppm** | **Abtötungsgrad in % nach 7^{d}** |
| Bsp. I-2-5 | 500 | 100 |
| Bsp. I-2-2 | 500 | 100 |
| Bsp. I-2-1 | 500 | 100 |
| Bsp. I-2-3 | 500 | 100 |

### Beispiel E

### Tetranychus-Test (OP-resistent/Tauchbehandlung)

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emugator und verdünnt das Konzentrat mit emulgatorhaltigem Wasser auf die gewünschte Konzentration.

Bohnenpflanzen (Phaseolus vulgaris), die stark von allen Stadien der Gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele gute Wirksamkeit:

**Tabelle E**

| pflanzenschädigende Milben | | |
|---|---|---|
| **Tetranychus-Test** (OP-resistent/Tauchbehandlung) | | |
| **Wirkstoffe** | **Wirkstoffkonzentration in ppm** | **Abtötungsgrad in % nach 7^{d}** |
| Bsp. I-2-5 | 100 | 98 |
| Bsp. I-2-4 | 100 | 80 |
| Bsp. I-1-1 | 100 | 100 |
| Bsp. I-2-2 | 100 | 98 |
| Bsp. I-2-1 | 100 | 100 |
| Bsp. I-2-3 | 100 | 100 |

### Beispiel F

### Plasmopara-Test (Rebe) / protektiv

| | |
|---|---|
| Lösungsmittel: | 24,5 Gewichtsteile Aceton |
| | 24,5 Gewichtsteile Dimethylacetamid |
| | |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von Plasmopara viticola inokuliert und verbleiben dann 1 Tag in einer Inkubationskabine bei ca. 20°C und 100 % relativer Luftfeuchtigkeit. Anschließend werden die Pflanzen 4 Tage im Gewächshaus bei ca. 21 °C und ca. 90 % Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle F**

| Plasmopara-Test (Rebe) / protektiv | | |
|---|---|---|
| Wirkstoff | Aufwandmenge an Wirkstoff in g/ha | % Wirkungsgrad |
| Bsp. I-1-1 | 100 | 100 |

### Beispiel G

### Sphaerotheca-Test (Gurke) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von Sphaerotheca fuliginea inokuliert. Die Pflanzen werden dann bei ca. 23°C und einer relativen Luftfeuchtigkeit von ca. 70 % im Gewächshaus aufgestellt.

7 Tage nach der Inokulation erfolgt die Auswertung. Die dabei 0 % ein Wirkungsgrad, der demjenigen der Kontrolle entspricht, während ein Wirkungsgrad von 100 % bedeutet, dass kein Befall beobachtet wird.

**Tabelle G**

| Sphaerothca-Test (Gurke) / protektiv | | |
|---|---|---|
| **Wirkstoff** | **Aufwandmenge an Wirkstoff in g/ha** | **% Wirkungsgrad** |
| Bsp. 1-2-1 | 100 | 97 |

### Beispiel H

### Venturia-Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle H**

| Venturia-Test (Apfel) / protektiv | | |
|---|---|---|
| **Wirkstoff** | **Aufwandmenge an Wirkstoff in g/ha** | **% Wirkungsgrad** |
| Bsp. I-1-1 | 100 | 100 |
| Bsp. I-2-1 | 100 | 73 |
| Bsp. I-2-3 | 100 | 86 |

### Beispiel I

### Botrytis-Test. (Bohne) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in den angegebenen Aufwandmenge besprüht. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten Kammer bei ca. 20°C und 100 % relativer Luftfeuchtigkeit aufgestellt.

2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, dass kein Befall beobachtet wird.

**Tabelle I**

| Botrytis - Test (Bohne) / protektiv | | |
|---|---|---|
| **Wirkstoff** | **Aufwandmenge an Wirkstoff in g/ha** | **% Wirkungsgrad** |
| Bsp. I-1-1 | 100 | - 96 |

### Beispiel J

### Phytophthora-Test (Tomate) / protektiv

| | |
|---|---|
| Lösungsmittel: | 49 Gewichtsteile N,N-Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Tomatenpflanzen mit der Wirkstoffzubereitung in der angegebenen Aufwandmenge besprüht. 1 Tag nach der Behandlung werden die Pflanzen mit einer Sporensuspension von Phytophthora infestans inokuliert und stehen dann 24 h bei 100 % relativer Feuchte und 20°C. Anschließend werden die Pflanzen in einer Klimazelle bei ca. 96 % relativer Luftfeuchtigkeit und einer Temperatur von ca. 20°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle J**

| Phytophthora-Test (Tomate) /protektiv | | |
|---|---|---|
| **Wirkstoff** | **Aufwandmenge an Wirkstoff in g/ha** | **Wirkungsgrad in %** |
| Bsp. I-2-1 | 750 | 89 |
| Bsp. I-1-3 | 750 | 80 |
| Bsp. I-1-4 | 750 | 90 |

### Beispiel K

### Post-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben, so dass die jeweils gewünschten Wirkstoffmengen pro Flächeneinheit ausgebracht werden. Die Konzentration der Spritzbrühe wird so gewählt, dass in 10001 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

### Beispiel L

### Pre-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung bespritzt, so dass die jeweils gewünschten Wirkstoffmengen pro Flächeneinheit ausgebracht werden. Die Konzentration der Spritzbrühe wird so gewählt, dass in 1000 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

| pre-emergence | g ai/ha | Zuckerrüben | Alopecurus | Echinochloa | Setaria |
|---|---|---|---|---|---|
| Bsp. I-1-1 | 250 | 0 | - | 100 | 90 |
| Bsp. I-2-2 | 250 | 0 | 90 | 95 | 90 |
| Bsp. I-2-1 | 250 | 0 | 70 | 90 | 80 |
| Bsp. I-2-3 | 250 | 0 | 80 | 80 | 90 |

### Beispiel M

### Grenzkonzentrations-Test / Bodeninsekten - Behandlung transgener Pflanzen

| | |
|---|---|
| Testinsekt: | Diabrotica balteata - Larven im Boden |
| | |
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/1) angegeben wird. Man füllt den Boden in 0,25 1 Töpfe und läßt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel N

### Heliothis virescens - Test - Behandlung transgener Pflanzen

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

## Patentansprüche

1. Verbindungen der Formel (I) 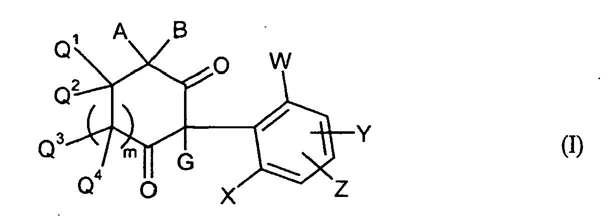 in welcher
W für Cyano, Halogen, Alkyl, Alkenyl, Alkinyl, Alkoxy, Halogenalkyl oder Halogenalkoxy steht,
X für Wasserstoff, Halogen, Alkyl, Alkoxy, Halogenalkyl, Halogenalkoxy oder Cyano steht,
Y für Wasserstoff, Halogen, Alkyl, Alkoxy, Halogenalkyl, Halogenalkoxy, Cyano oder gegebenenfalls substituiertes Phenyl steht,
Z für Wasserstoff, Halogen, Alkyl, Alkoxy, Halogenalkyl, Halogenalkoxy oder Cyano steht,
G für Halogen oder Nitro steht,
m für die Zahlen 0 oder 1 steht,
A für Wasserstoff, jeweils gegebenenfalls durch Halogen substituiertes Alkyl, Alkenyl, Alkoxyalkyl, Polyalkoxyalkyl, Alkylthioalkyl, gesättigtes oder ungesättigtes, gegebenenfalls substituiertes Cycloalkyl, in welchem gegebenenfalls mindestens ein Ringatom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
B für Wasserstoff oder Alkyl steht,
A und B gemeinsam mit dem Kohlenstoffatom an das sie gebunden sind, für einen gesättigten oder ungesättigten, gegebenenfalls mindestens ein Heteroatom enthaltenden unsubstituierten oder substituierten Cyclus stehen,
A und Q¹ gemeinsam für gegebenenfalls substituiertes Alkandiyl stehen, in welchem zwei nicht direkt benachbarte Kohlenstoffatome gegebenenfalls einen weiteren gegebenenfalls substituierten Cyclus bilden,
Q¹ für Wasserstoff, Alkyl, Alkoxyalkyl, gegebenenfalls substituiertes Cycloalkyl (worin gegebenenfalls eine Methylengruppe durch Sauerstoff oder Schwefel ersetzt ist) oder jeweils gegebenenfalls substituiertes Phenyl, Hetaryl, Phenylalkyl oder Hetarylalkyl steht,
Q², Q³, Q⁴ unabhängig voneinander für Wasserstoff oder Alkyl steht,
Q¹ und Q² gemeinsam mit dem Kohlenstoffatom, an das sie gebunden sind, für einen gesättigten oder ungesättigten gegebenenfalls ein Heteroatom enthaltenden unsubstituierten oder substituierten Cyclus stehen.

2. Verbindungen der Formel (I), gemäß Anspruch 1, in welcher
W für Halogen, C₁-C₆-Alkyl, C₂-C₆-Alkenyl, C₂-C₆-Alkinyl, C₁-C₆-Alkoxy, C₁-C₄-Halogenalkyl, C₁-C₄-Halogenalkoxy oder Cyano steht,
X für Wasserstoff, Halogen, C₁-C₆-Alkyl, C₁-C₆-Alkoxy, C₁-C₄-Halogenalkyl, C₁-C₄-Halogenalkoxy oder Cyano steht,
Y für Wasserstoff, Halogen, C₁-C₆-Alkyl, C₁-C₆-Alkoxy, C₁-C₄-Halogenalkyl, C₁-C₄-Halogenalkoxy, Cyano oder für die Gruppe 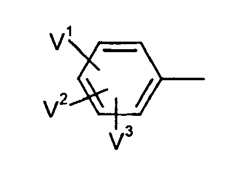 steht,
V¹ für Wasserstoff, Halogen, C₁-C₁₂-Alkyl, C₁-C₆-Alkoxy, C₁-C₆-Alkylthio, C₁-C₆-Alkylsulfinyl, C₁-C₆-Alkylsulfonyl, C₁-C₄-Halogenalkyl, C₁-C₄-Halogenalkoxy, Nitro, Cyano oder jeweils gegebenenfalls einfach oder mehrfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio steht,
V² und V³ unabhängig voneinander für Wasserstoff, Halogen, C₁-C₆-Alkyl, C₁-C₆-Alkoxy, C₁-C₄-Halogenalkyl oder C₁-C₄-Halogenalkoxy stehen,
V¹ und V² zusammen gemeinsam mit den Kohlenstoffatomen, an die sie gebunden sind für einen gegebenenfalls durch C₁-C₄-Alkyl oder Halogen substituierten 5- oder 6-gliedrigen Cyclus stehen, in welchem gegebenenfalls ein bis zwei Kohlenstoffatome durch Sauerstoff, Schwefel oder Stickstoff ersetzt sein können,
Z für Wasserstoff, Halogen, C₁-C₆-Alkyl, C₁-C₆-Alkoxy, C₁-C₄-Halogenalkyl, C₁-C₄-Halogenalkoxy oder Cyano steht,
G für Halogen oder Nitro steht,
m für die Zahlen 0 oder 1 steht,
A für Wasserstoff oder jeweils gegebenenfalls durch Halogen substituiertes C₁-C₁₂-Alkyl, C₃-C₈-Alkenyl, C₁-C₆-Alkoxy-C₁-C₄-alkyl, jeweils gegebenenfalls durch Halogen, C₁-C₄-Alkyl oder C₁-C₄-Alkoxy substituiertes C₃-C₈-Cycloalkyl oder C₃-C₆-Cycloalkyl-C₁-C₄-alkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Benzyl, Hetaryl mit 5 bis 6 Ringatomen oder Hetaryl-C₁-C₄-alkyl mit 5 bis 6 Ringatomen steht,
B für Wasserstoff oder C₁-C₆-Alkyl steht,
A, B und das Kohlenstoffatom an das sie gebunden sind, für gesättigtes C₃-C₁₀-Cycloalkyl oder ungesättigtes C₅-C₁₀-Cycloalkyl stehen, worin gegebenenfalls ein Ringglied durch Sauerstoff oder Schwefel ersetzt ist und welche gegebenenfalls einfach oder zweifach durch C₁-C₆-Alkyl, C₃-C₈-Cycloalkyl, C₁-C₆-Halogenalkyl, C₁-C₆-Alkoxy, C₁-C₆-Alkylthio, Halogen oder Phenyl substituiert sind,
A und Q¹ gemeinsam für gegebenenfalls einfach oder zweifach, gleich oder verschieden durch C₁-C₄-Alkyl, C₁-C₄-Alkoxy substituiertes C₃-C₆-Alkandiyl stehen,
Q¹ für Wasserstoff, C₁-C₆-Alkyl, C₁-C₆-Alkoxy-C₁-C₂-alkyl, gegebenenfalls durch Fluor, Chlor, C₁-C₄-Alkyl, C₁-C₂-Halogenalkyl oder C₁-C₄-Alkoxy substituiertes C₃-C₈-Cycloalkyl, worin gegebenenfalls eine Methylengruppe durch Sauerstoff oder Schwefel ersetzt ist oder jeweils gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenyl, Pyridyl, Thienyl, Thiazolyl, Phenyl-C₁-C₄-alkyl, Pyridyl-C₁-C₂-alkyl oder Thiazolyl-C₁-C₂-alkyl steht,
Q², Q³, Q⁴ unabhängig voneinander für Wasserstoff oder C₁-C₄-Alkyl stehen,
Q¹ und Q² gemeinsam mit dem Kohlenstoffatom, an das sie gebunden sind, für gegebenenfalls durch C₁-C₆-Alkyl, C₁-C₆-Alkoxy oder C₁-C₂-Halogenalkyl substituiertes C₃-C₇-Cycloalkyl stehen, in welchem gegebenenfalls ein Ringglied durch Sauerstoff oder Schwefel ersetzt ist.

3. Verbindungen der Formel (I) gemäß Anspruch 1, in welcher
W für Fluor, Chlor, Brom, C₁-C₄-Alkyl, C₁-C₄-Alkoxy, C₁-C₂-Halogenalkyl, C₁-C₂-Halogenalkoxy oder Cyano steht,
X für Wasserstoff, Fluor, Chlor, Brom, C₁-C₄-Alkyl oder C₁-C₄-Alkoxy steht,
Y für Wasserstoff, Fluor, Chlor, Brom, C₁-C₆-Alkyl, C₁-C₆-Alkoxy, C₁-C₂-Halogenalkyl, C₁-C₂-Halogenalkoxy, Cyano oder für die Gruppe 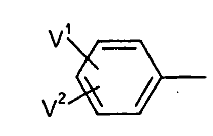 steht,
V¹ für Wasserstoff, Fluor, Chlor, Brom, C₁-C₆-Alkyl, C₁-C₄-Alkoxy, C₁-C₂-Halogenalkyl, C₁-C₂-Halogenalkoxy, Nitro, Cyano oder jeweils gegebenenfalls einfach oder zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl oder Phenoxy steht,
V² für Wasserstoff, Fluor, Chlor, C₁-C₄-Alkyl, C₁-C₄-Alkoxy, C₁-C₂-Halogenalkyl oder C₁-C₂-Halogenalkoxy steht,
V¹ und V² zusammen gemeinsam mit den Kohlenstoffatomen, an die sie gebunden sind, für einen gegebenenfalls durch Fluor oder Methyl substituierten 5- oder 6-gliedrigen Cyclus stehen, in welchem gegebenenfalls ein bis zwei Kohlenstoffatome durch Sauerstoff ersetzt sein können,
Z für Wasserstoff, Fluor, Chlor, Brom, C₁-C₄-Alkyl, C₁-C₄-Alkoxy, C₁-C₂-Halogenalkyl, C₁-C₂-Halogenalkoxy oder Cyano steht,
G für Chlor, Brom oder Nitro steht,
m für die Zahlen 0 oder 1 steht,
A für Wasserstoff, für jeweils gegebenenfalls durch Fluor substituiertes C₁-C₈-Alkyl oder C₁-C₄-Alkoxy-C₁-C₂-alkyl, für jeweils gegebenenfalls durch Fluor, Chlor, Methyl, Ethyl oder Methoxy substituiertes C₅-C₆-Cycloalkyl oder C₃-C₆-Cycloalkyl-C₁-C₂-alkyl, in welchem gegebenenfalls ein Ringglied durch Sauerstoff oder Schwefel ersetzt ist oder jeweils gegebenenfalls durch Fluor, Chlor, Brom, C₁-C₄-Alkyl, C₁-C₂-Halogenalkyl, C₁-C₄-Alkoxy oder C₁-C₂-Halogenalkoxy substituiertes Phenyl oder Benzyl steht,
B für Wasserstoff oder C₁-C₄-Alkyl steht,
A, B und das Kohlenstoffatom an das sie gebunden sind, für gesättigtes C₅-C₇-Cycloalkyl stehen, worin gegebenenfalls ein Ringglied durch Sauerstoff ersetzt ist und welches gegebenenfalls einfach durch C₁-C₄-Alkyl, Trifluormethyl oder C₁-C₄-Alkoxy substituiert ist,
mit der Maßgabe, dass dann Q¹ nur für Wasserstoff steht,
A und Q¹ gemeinsam für gegebenenfalls einfach oder zweifach durch Methyl, Ethyl, Methoxy oder Ethoxy substitutiertes C₃-C₄-Alkandiyl stehen,
Q¹ für Wasserstoff, C₁-C₆-Alkyl, C₁-C₄-Alkoxy-C₁-C₂-alkyl, oder gegebenenfalls durch Methyl oder Methoxy substituiertes C₃-C₆-Cycloalkyl, worin gegebenenfalls eine Methylengruppe durch Sauerstoff ersetzt ist, oder jeweils gegebenenfalls durch Fluor, Chlor, Brom, C₁-C₄-Alkyl, C₁-C₄-Alkoxy, Trifluormethyl oder Trifluormethoxy substituiertes Phenyl oder Benzyl steht,
Q², Q³, Q⁴ unabhängig voneinander für Wasserstoff, Methyl oder Ethyl stehen,
Q¹ und Q² gemeinsam mit dem Kohlenstoff, an das sie gebunden sind, für gegebenenfalls durch C₁-C₄-Alkyl oder C₁-C₄-Alkoxy substituiertes gesättigtes C₅-C₆-Cycloalkyl stehen, in welchem gegebenenfalls ein Ringglied durch Sauerstoff ersetzt ist,
mit der Maßgabe, dass dann A nur für Wasserstoff steht.

4. Verbindungen der Formel (I) gemäß Anspruch 1, in welcher
W für Chlor, Brom, Methyl, Ethyl, Propyl, Methoxy, Ethoxy, Trifluormethyl, Difluormethoxy, Trifluormethoxy oder Cyano steht,
X für Wasserstoff, Chlor, Brom, Methyl, Ethyl, Propyl, Methoxy oder Ethoxy steht,
Y für Wasserstoff, Fluor, Chlor, Brom, Methyl, Ethyl, n-Propyl, i-Butyl, CH(CH₃)-i-Butyl, Methoxy, Ethoxy, Trifluormethyl, Trifluormethoxy, Difluormethoxy, Cyano oder für die Gruppe 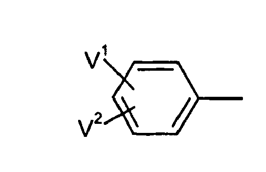 steht,
V¹ für Wasserstoff, Fluor, Chlor, Brom, Methyl, Ethyl, n-Propyl, iso-Propyl, n-Butyl, iso-Butyl, tert.-Butyl, Methoxy, Ethoxy, n-Propoxy, iso-Propoxy, Trifluormethyl oder Trifluormethoxy steht,
V² für Wasserstoff, Fluor, Chlor, Methyl, Ethyl, n-Propyl, iso-Propyl, Methoxy, Ethoxy oder Trifluormethyl steht,
Z für Wasserstoff, Fluor, Chlor, Brom, Methyl, Ethyl, Propyl, Methoxy, Ethoxy, Trifluormethyl, Trifluormethoxy, Difluormethoxy oder Cyano steht,
G für Chlor, Brom oder Nitro steht,
m für die Zahlen 0 oder 1 steht,
A für Wasserstoff, Methyl, Ethyl, n-Propyl, iso-Propyl, n-Butyl, iso-Butyl, Methoxymethyl oder Ethoxymethyl steht,
B für Wasserstoff, Methyl oder Ethyl steht,
A, B und das Kohlenstoffatom, an das sie gebunden sind, für gesättigtes C₅-C₇-Cycloalkyl stehen, in welchem gegebenenfalls ein Ringglied durch Sauerstoff ersetzt ist und welches gegebenenfalls einfach durch Methyl, Ethyl, Isopropyl, Trifluormethyl, Methoxy, Ethoxy, n-Propoxy, n-Butoxy oder Isobutoxy substituiert ist,
mit der Maßgabe, dass dann Q¹, Q², Q³ und Q⁴ nur für Wasserstoff stehen,
A und Q¹ gemeinsam für C₃-C₄-Alkandiyl stehen,
Q¹ für Wasserstoff, Methyl, Ethyl, n-Propyl, iso-Propyl, n-Hexyl, Cyclopropyl, Cyclopentyl oder Cyclohexyl steht,
Q², Q³, Q⁴ unabhängig voneinander für Wasserstoff, Methyl oder Ethyl stehen,
Q¹ und Q² gemeinsam mit dem Kohlenstoff, an den sie gebunden sind, für gegebenenfalls durch Methyl, Ethyl, Methoxy, Ethoxy, n-Propoxy oder n-Butoxy substituiertes gesättigtes C₅-C₆-Cycloalkyl stehen, in welchem gegebenenfalls ein Ringglied durch Sauerstoff ersetzt ist,
mit der Maßgabe, dass dann A, B, Q³ und Q⁴ nur für Wasserstoff stehen.

5. Verbindungen der Formel (I) gemäß Anspruch 1, in welcher
W für Methyl oder Chlor steht,
Y für gegebenenfalls einfach bis zweifach durch Chlor oder Fluor substituiertes Phenyl, für Chlor, Brom, Methyl, Ethyl, n-Propyl, i-Butyl, CH(CH₃)-i-Butyl oder Trifluormethyl steht,
Z für Wasserstoff steht,
G für Chlor steht,
m für 0 oder 1 steht,
A für Wasserstoff oder Methyl steht,
B für Wasserstoff oder Methyl steht,
A, B und das Kohlenstoffatom, an das sie gebunden sind für C₅-C₇-Cycloalkyl stehen, mit der Maßgabe, dass dann Q¹ und Q² nur für Wasserstoff stehen,
Q¹ für Wasserstoff, Methyl, Ethyl, i-Propyl oder n-Hexyl stehen,
Q² für Wasserstoff oder Methyl stehen,
Q¹, Q² und das Kohlenstoffatom, an das sie gebunden sind für C₅-C₆-Cycloalkyl stehen, mit der Maßgabe, dass dann A und B nur für Wasserstoff stehen,
Q³ für Wasserstoff steht,
Q⁴ für Wasserstoff steht.

6. Verfahren zur Herstellung von Verbindungen der Formel (I) gemäß Anspruch 1, **dadurch gekennzeichnet, dass** man zum Erhalt von
A) Verbindungen der Formeln (I-I) bis (1-2) 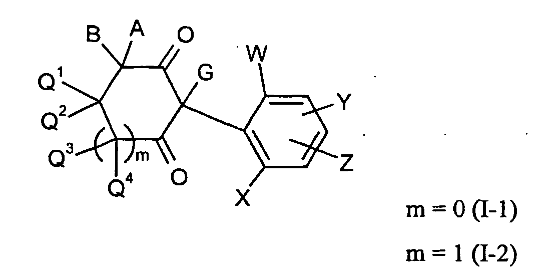 in welcher
A, B, Q¹, Q², Q³, Q⁴, W, X, Y und Z die oben angegebene Bedeutung haben
und
G für Halogen steht,
Verbindungen der Formeln (II-1) bis (II-2) 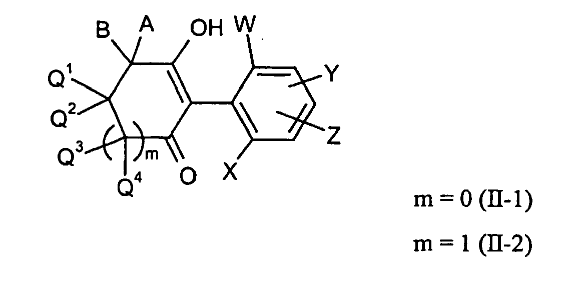 in welcher
A, B, Q¹, Q², Q³, Q⁴, W, X, Y und Z die oben angegebene Bedeutung haben
mit Halogenierungsmitteln in Gegenwart eines Lösungsmittels und gegebenenfalls in Gegenwart eines Radikalstarters umsetzt und
B) Verbindungen der Formeln (I-1) bis (I-2), 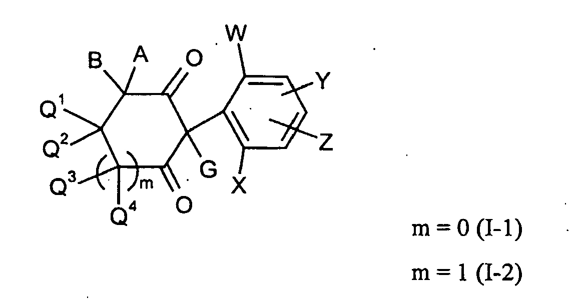 in welcher
A, B, Q¹,Q², Q³, Q⁴, W, X, Y und Z die oben angegebene Bedeutung haben
und
G für Nitro steht,
Verbindungen der Formeln (II-1) bis (II-2) 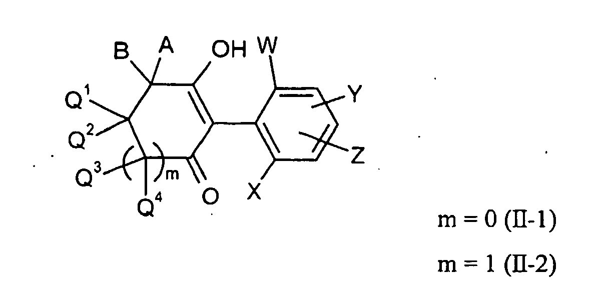 in welcher
A, B, Q¹, Q², Q³, Q⁴, W, X, Y und Z die oben angegebene Bedeutung haben,
mit Nitrierungsreagenzien wie z.B. rauchende Salpetersäure in Gegenwart eines Lösungsmittels umsetzt.

7. Mittel zur Bekämpfung von Schädlingen, unerwünschtem Pflanzenwuchs und/oder unerwünschten Mikroorganismen, **gekennzeichnet durch** einen Gehalt an mindestens einer Verbindung der Formel (I) gemäß Anspruch 1.

8. Verfahren zur Bekämpfung von tierischen Schädlingen, unerwünschtem Pflanzenbewuchs und/oder unerwünschten Mikroorganismen, **dadurch gekennzeichnet, dass** man Verbindungen der Formel (I) gemäß Anspruch 1 auf Schädlinge, unerwünschtem Pflanzenwuchs, unerwünschte Mikroorganismen und/oder ihren Lebensraum einwirken läßt.

9. Verwendung von Verbindungen der Formel (I) gemäß Anspruch 1 zur Bekämpfung von tierischen Schädlingen, unerwünschtem Pflanzenbewuchs und/oder unerwünschten Mikroorganismen.

10. Verfahren zur Herstellung von Mitteln zur Bekämpfung von Schädlingen, unerwünschtem Pflanzenwuchs und/oder unerwünschten Mikroorganismen, **dadurch gekennzeichnet, dass** man Verbindungen der Formel (I) gemäß Anspruch 1 mit Streckmitteln und/oder oberflächenaktiven Stoffen vermischt.

11. Verwendung von Verbindungen der Formel (I) gemäß Anspruch 1 zur Herstellung von Mitteln zur Bekämpfung von Schädlingen, unerwünschtem Pflanzenwuchs und/oder unerwünschten. Mikroorganismen.

## Claims

1. Compounds of the formula (I) 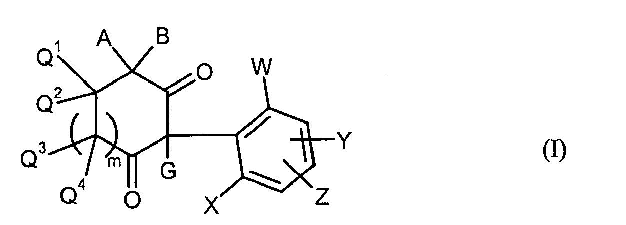 in which
W represents cyano, halogen, alkyl, alkenyl, alkynyl, alkoxy, haloalkyl or haloalkoxy,
X represents hydrogen, halogen, alkyl, alkoxy, haloalkyl, haloalkoxy or cyano,
Y represents hydrogen, halogen, alkyl, alkoxy, haloalkyl, haloalkoxy, cyano or optionally substituted phenyl,
Z represents hydrogen, halogen, alkyl, alkoxy, haloalkyl, haloalkoxy or cyano,
G represents halogen or nitro,
m represents the number 0 or 1,
A represents hydrogen, in each case optionally halogen-substituted alkyl, alkenyl, alkoxyalkyl, polyalkoxyalkyl, alkylthioalkyl, saturated or unsaturated, optionally substituted cycloalkyl in which optionally at least one ring atom is replaced by a heteroatom, or in each case optionally halogen-, alkyl-, haloalkyl-, alkoxy-, haloalkoxy-, cyano- or nitro-substituted aryl, arylalkyl or hetaryl,
B represents hydrogen or alkyl,
A and B together with the carbon atom to which they are attached represent a saturated or unsaturated, unsubstituted or substituted cycle which optionally contains at least one heteroatom,
A and Q¹ together represent optionally substituted alkanediyl in which optionally two not directly adjacent carbon atoms form a further optionally substituted cycle,
Q¹ represents hydrogen, alkyl, alkoxyalkyl, optionally substituted cycloalkyl (in which optionally one methylene group is replaced by oxygen or sulphur) or in each case optionally substituted phenyl, hetaryl, phenylalkyl or hetarylalkyl,
Q², Q³, Q⁴ independently of one another represent hydrogen or alkyl,
Q¹ and Q² together with the carbon atom to which they are attached represent a saturated or unsaturated, unsubstituted or substituted cycle which optionally contains a heteroatom.

2. Compounds of the formula (I) according to Claim 1 in which
W represents halogen, C₁-C₆-alkyl, C₂-C₆-alkenyl, C₂-C₆-alkynyl, C₁-C₆-alkoxy, C₁-C₄-haloalkyl, C₁-C₄-haloalkoxy or cyano,
X represents hydrogen, halogen, C₁-C₆-alkyl, C₁-C₆-alkoxy, C₁-C₄-haloalkyl, C₁-C₄-haloalkoxy or cyano,
Y represents hydrogen, halogen, C₁-C₆-alkyl, C₁-C₆-alkoxy, C₁-C₄-haloalkyl, C₁-C₄-haloalkoxy, cyano or represents the group 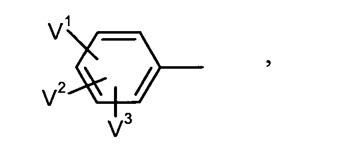
V¹ represents hydrogen, halogen, C₁-C₁₂-alkyl, C₁-C₆-alkoxy, C₁-C₆-alkylthio, C₁-C₆-alkylsulphinyl, C₁-C₆-alkylsulphonyl, C₁-C₄-haloalkyl, C₁-C₄-haloalkoxy, nitro, cyano or represents phenyl, phenoxy, phenoxy-C₁-C₄-alkyl, phenyl-C₁-C₄-alkoxy, phenylthio-C₁-C₄-alkyl or phenyl-C₁-C₄-alkylthio, each of which is optionally mono-or polysubstituted by halogen, C₁-C₆-alkyl, C₁-C₆-alkoxy, C₁-C₄-haloalkyl, C₁-C₄-haloalkoxy, nitro or cyano,
V² and V³ independently of one another represent hydrogen, halogen, C₁-C₆-alkyl, C₁-C₆-alkoxy, C₁-C₄-haloalkyl or C₁-C₄-haloalkoxy,
V¹ and V² together and together with the carbon atoms to which they are attached represent an optionally C₁-C₄-alkyl- or halogen-substituted 5-or 6-membered cycle in which optionally one or two carbon atoms may be replaced by oxygen, sulphur or nitrogen,
Z represents hydrogen, halogen, C₁-C₆-alkyl, C₁-C₆-alkoxy, C₁-C₄-haloalkyl, C₁-C₄-haloalkoxy or cyano,
G represents halogen or nitro,
m represents the number 0 or 1,
A represents hydrogen or in each case optionally halogen-substituted C₁-C₁₂-alkyl, C₃-C₈-alkenyl, C₁-C₆-alkoxy-C₁-C₄-alkyl, in each case optionally halogen-, C₁-C₄-alkyl- or C₁-C₄-alkoxy-substituted C₃-C₈-cycloalkyl or C₃-C₆-cycloalkyl-C₁-C₄-alkyl in which optionally one or two not directly adjacent ring members are replaced by oxygen and/or sulphur or represent in each case optionally halogen-, C₁-C₆-alkyl-, C₁-C₆-haloalkyl-, C₁-C₆-alkoxy-, C₁-C₆-haloalkoxy-, cyano- or nitro-substituted phenyl, benzyl, hetaryl having 5 or 6 ring atoms or hetaryl-C₁-C₄-alkyl having 5 or 6 ring atoms,
B represents hydrogen or C₁-C₆-alkyl,
A, B and the carbon atom to which they are attached represent saturated C₃-C₁₀-cycloalkyl or unsaturated C₅-C₁₀-cycloalkyl in which optionally one ring member is replaced by oxygen or sulphur and which are optionally mono- or disubstituted by C₁-C₆-alkyl, C₃-C₈-cycloalkyl, C₁-C₆-haloalkyl, C₁-C₆-alkoxy, C₁-C₆-alkylthio, halogen or phenyl,
A and Q¹ together represent C₃-C₆-alkanediyl which is optionally mono- or disubstituted by identical or different substituents from the group consisting of C₁-C₄-alkyl and C₁-C₄-alkoxy,
Q¹ represents hydrogen, C₁-C₆-alkyl, C₁-C₆-alkoxy-C₁-C₂-alkyl, optionally fluorine-, chlorine-, C₁-C₄-alkyl-, C₁-C₂-haloalkyl- or C₁-C₄-alkoxy-substituted C₃-C₈-cycloalkyl in which optionally one methylene group is replaced by oxygen or sulphur or in each case optionally halogen-, C₁-C₄-alkyl-, C₁-C₄-alkoxy-, C₁-C₂-haloalkyl-, C₁-C₂-haloalkoxy-, cyano- or nitro-substituted phenyl, pyridyl, thienyl, thiazolyl, phenyl-C₁-C₄-alkyl, pyridyl-C₁-C₂-alkyl or thiazolyl-C₁-C₂-alkyl,
Q², Q³, Q⁴ independently of one another represent hydrogen or C₁-C₄-alkyl,
Q¹ and Q² together with the carbon atom to which they are attached represent optionally C₁-C₆-alkyl-, C₁-C₆-alkoxy- or C₁-C₂-haloalkyl-substituted C₃-C₇-cycloalkyl in which optionally one ring member is replaced by oxygen or sulphur.

3. Compounds of the formula (I) according to Claim 1 in which
W represents fluorine, chlorine, bromine, C₁-C₄-alkyl, C₁-C₄-alkoxy, C₁-C₂-haloalkyl, C₁-C₂-haloalkoxy or cyano,
X represents hydrogen, fluorine, chlorine, bromine, C₁-C₄-alkyl or C₁-C₄-alkoxy,
Y represents hydrogen, fluorine, chlorine, bromine, C₁-C₆-alkyl, C₁-C₆-alkoxy, C₁-C₂-haloalkyl, C₁-C₂-haloalkoxy, cyano or represents the group 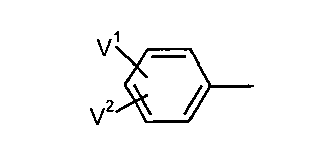
V¹ represents hydrogen, fluorine, chlorine, bromine, C₁-C₆-alkyl, C₁-C₄-alkoxy, C₁-C₂-haloalkyl, C₁-C₂-haloalkoxy, nitro, cyano or represents phenyl or phenoxy, each of which is optionally mono- or disubstituted by fluorine, chlorine, bromine, C₁-C₄-alkyl, C₁-C₄-alkoxy, C₁-C₂-haloalkyl, C₁-C₂-haloalkoxy, nitro or cyano,
V² represents hydrogen, fluorine, chlorine, C₁-C₄-alkyl, C₁-C₄-alkoxy, C₁-C₂-haloalkyl or C₁-C₂-haloalkoxy,
V¹ and V² together and together with the carbon atoms to which they are attached represent an optionally fluorine- or methyl-substituted 5- or 6-membered cycle in which optionally one or two carbon atoms may be replaced by oxygen,
Z represents hydrogen, fluorine, chlorine, bromine, C₁-C₄-alkyl, C₁-C₄-alkoxy, C₁-C₂-haloalkyl, C₁-C₂-haloalkoxy or cyano,
G represents chlorine, bromine or nitro,
m represents the number 0 or 1,
A represents hydrogen, represents in each case optionally fluorine-substituted C₁-C₈-alkyl or C₁-C₄-alkoxy-C₁-C₂-alkyl, represents in each case optionally fluorine-, chlorine-, methyl-, ethyl- or methoxy-substituted C₅-C₆-cycloalkyl or C₃-C₆-cycloalkyl-C₁-C₂-alkyl in which optionally one ring member is replaced by oxygen or sulphur or in each case optionally fluorine-, chlorine-, bromine-, C₁-C₄-alkyl-, C₁-C₂-haloalkyl-, C₁-C₄-alkoxy- or C₁-C₂-haloalkoxy-substituted phenyl or benzyl,
B represents hydrogen or C₁-C₄-alkyl,
A, B and the carbon atom to which they are attached represent saturated C₅-C₇-cycloalkyl in which optionally one ring member is replaced by oxygen and which is optionally monosubstituted by C₁-C₄-alkyl, trifluoromethyl or C₁-C₄-alkoxy,
with the proviso that in this case Q¹ only represents hydrogen,
A and Q¹ together represent C₃-C₄-alkanediyl which is optionally mono- or disubstituted by methyl, ethyl, methoxy or ethoxy,
Q¹ represents hydrogen, C₁-C₆-alkyl, C₁-C₄-alkoxy-C₁-C₂-alkyl or optionally methyl- or methoxy-substituted C₃-C₆-cycloalkyl in which optionally one methylene group is replaced by oxygen, or in each case optionally fluorine-, chlorine-, bromine-, C₁-C₄-alkyl-, C₁-C₄-alkoxy-, trifluoromethyl- or trifluoromethoxy-substituted phenyl or benzyl,
Q², Q³, Q⁴ independently of one another represent hydrogen, methyl or ethyl,
Q¹ and Q² together with the carbon to which they are attached represent optionally C₁-C₄-alkyl- or C₁-C₄-alkoxy-substituted saturated C₅-C₆-cycloalkyl in which optionally one ring member is replaced by oxygen,
with the proviso that in this case A only represents hydrogen.

4. Compounds of the formula (I) according to Claim 1 in which
W represents chlorine, bromine, methyl, ethyl, propyl, methoxy, ethoxy, trifluoromethyl, difluoromethoxy, trifluoromethoxy or cyano,
X represents hydrogen, chlorine, bromine, methyl, ethyl, propyl, methoxy or ethoxy,
Y represents hydrogen, fluorine, chlorine, bromine, methyl, ethyl, n-propyl, i-butyl, CH(CH₃)-i-butyl, methoxy, ethoxy, trifluoromethyl, trifluoromethoxy, difluoromethoxy, cyano or represents the group 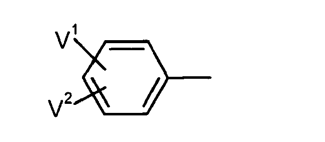
V¹ represents hydrogen, fluorine, chlorine, bromine, methyl, ethyl, n-propyl, isopropyl, n-butyl, isobutyl, tert-butyl, methoxy, ethoxy, n-propoxy, isopropoxy, trifluoromethyl or trifluoromethoxy,
V² represents hydrogen, fluorine, chlorine, methyl, ethyl, n-propyl, isopropyl, methoxy, ethoxy or trifluoromethyl,
Z represents hydrogen, fluorine, chlorine, bromine, methyl, ethyl, propyl, methoxy, ethoxy, trifluoromethyl, trifluoromethoxy, difluoromethoxy or cyano,
G represents chlorine, bromine or nitro,
m represents the number 0 or 1,
A represents hydrogen, methyl, ethyl, n-propyl, isopropyl, n-butyl, isobutyl, methoxymethyl or ethoxymethyl,
B represents hydrogen, methyl or ethyl,
A, B and the carbon atom to which they are attached represent saturated C₅-C₇-cycloalkyl in which optionally one ring member is replaced by oxygen and which is optionally monosubstituted by methyl, ethyl, isopropyl, trifluoromethyl, methoxy, ethoxy, n-propoxy, n-butoxy or isobutoxy,
with the proviso that in this case Q¹, Q², Q³ and Q⁴ only represent hydrogen,
A and Q¹ together represent C₃-C₄-alkanediyl,
Q¹ represents hydrogen, methyl, ethyl, n-propyl, isopropyl, n-hexyl, cyclopropyl, cyclopentyl or cyclohexyl,
Q², Q³, Q⁴ independently of one another represent hydrogen, methyl or ethyl,
Q¹ and Q² together with the carbon to which they are attached represent optionally methyl-, ethyl-, methoxy-, ethoxy-, n-propoxy- or n-butoxy-substituted saturated C₅-C₆-cycloalkyl in which optionally one ring member is replaced by oxygen,
with the proviso that in this case A, B, Q³ and Q⁴ only represent hydrogen.

5. Compounds of the formula (I) according to Claim 1 in which
W represents methyl or chlorine,
Y represents phenyl which is optionally mono- or disubstituted by fluorine or chlorine, represents chlorine, bromine, methyl, ethyl, n-propyl, i-butyl, CH(CH₃)-i-butyl or trifluoromethyl,
Z represents hydrogen,
G represents chlorine,
m represents 0 or 1,
A represents hydrogen or methyl,
B represents hydrogen or methyl,
A, B and the carbon atom to which they are attached represent C₅-C₇-cycloalkyl, with the proviso that in this case Q¹ and Q² only represent hydrogen,
Q¹ represents hydrogen, methyl, ethyl, i-propyl or n-hexyl,
Q² represents hydrogen or methyl,
Q¹, Q² and the carbon atom to which they are attached represent C₅-C₆-cycloalkyl, with the proviso that in this case A and B only represent hydrogen,
Q³ represents hydrogen,
Q⁴ represents hydrogen.

6. Process for preparing compounds of the formula (I) according to Claim 1, **characterized in that**, to obtain
A) compounds of the formulae (I-1) to (I-2) 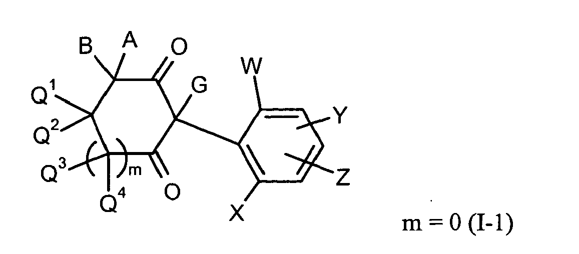 in which
A, B, Q¹, Q², Q³, Q⁴, W, X, Y and Z are as defined above
and
G represents halogen,
compounds of the formulae (II-1) to (II-2) 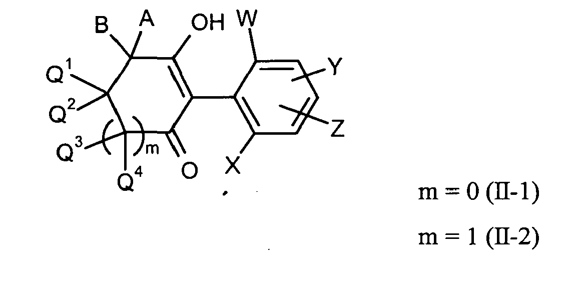 in which
A, B, Q¹, Q², Q³, Q⁴, W, X, Y and Z are as defined above
are reacted with halogenating agents in the presence of a solvent and, if appropriate, in the presence of a free-radical initiator and
B) compounds of the formulae (I-1) to (I-2) 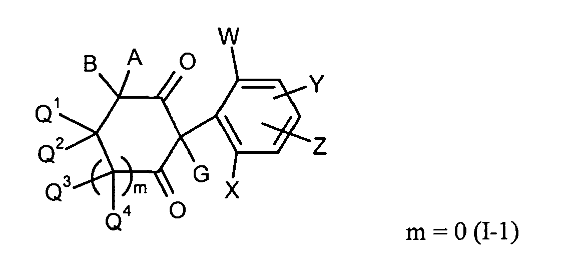 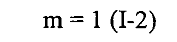 in which
A, B, Q¹, Q², Q³, Q⁴, W, X, Y and Z are as defined above
and
G represents nitro,
compounds of the formulae (II-1) to (II-2) 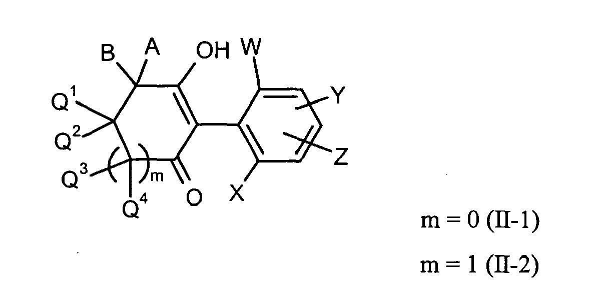 in which
A, B, Q¹, Q², Q³, Q⁴, W, X, Y and Z are as defined above
are reacted with nitrating agents, such as, for example, fuming nitric acid in the presence of a solvent.

7. Compositions for controlling pests, unwanted vegetation and/or unwanted microorganisms, **characterized in that** they comprise at least one compound of the formula (I) according to Claim 1.

8. Method for controlling animal pests, unwanted vegetation and/or unwanted microorganisms, **characterized in that** compounds of the formula (I) according to Claim 1 are allowed to act on pests, unwanted vegetation, unwanted microorganisms and/or their habitat.

9. Use of compounds of the formula (I) according to Claim 1 for controlling animal pests, unwanted vegetation and/or unwanted microorganisms.

10. Process for preparing compositions for controlling pests, unwanted vegetation and/or unwanted microorganisms, **characterized in that** compounds of the formula (I) according to Claim 1 are mixed with extenders and/or surfactants.

11. Use of compounds of the formula (I) according to Claim 1 for preparing compositions for controlling pests, unwanted vegetation and/or unwanted microorganisms.

## Revendications

1. Composés de la formule (I) : 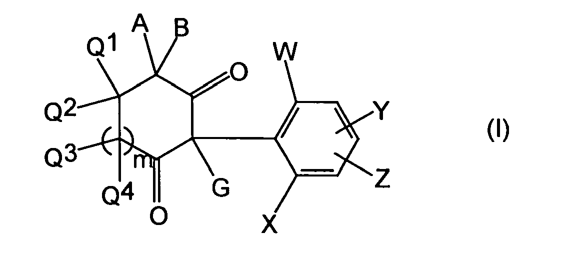 dans laquelle :
W représente un radical cyano, un atome d'halogène, un radical alcoyle, alcényle, alcynyle, alcoxy, halogénoalcoyle ou halogénoalcoxy,
X représente l'atome d'hydrogène, d'halogène, un radical alcoyle, alcoxy, halogénoalcoyle, halogénoalcoxy ou cyano,
Y représente l'atome d'hydrogène, d'halogène, un radical alcoyle, alcoxy, halogénoalcoyle, halogénoalcoxy, cyano ou phényle le cas échéant substitué,
Z représente l'atome d'hydrogène, d'halogène, un radical alcoyle, alcoxy, halogénoalcoyle, halogénoalcoxy ou cyano,
G représente un atome d'halogène ou le radical nitro,
m représente le nombre 0 ou 1,
A représente l'atome d'hydrogène, un radical alcoyle, alcényle, alcoxyalcoyle, polyalcoxyalcoyle, alcoylthioalcoyle, chaque fois le cas échéant substitué par un ou plusieurs atomes d'halogène, un radical cycloalcoyle saturé ou insaturé, le cas échéant substitué, dans lequel au moins un atome cyclique est le cas échéant, remplacé par un hétéroatome, ou un radical aryle, arylalcoyle ou hétaryle, chaque fois le cas échéant substitué par un ou plusieurs atomes d'halogène, un ou plusieurs radicaux alcoyle, halogénoalcoyle, alcoxy, halogénoalcoxy, cyano ou nitro,
B représente l'atome d'hydrogène ou un radical alcoyle,
A et B avec l'atome de carbone sur lequel ils sont liés, représentent un cycle saturé ou insaturé, contenant le cas échéant au moins un hétéroatome, non substitué ou substitué,
A et Q¹ ensemble, représentent un radical alcanediyle le cas échéant substitué, dans lequel deux atomes de carbone non directement voisins forment le cas échéant, un autre cycle le cas échéant substitué ;
Q¹ représente l'atome d'hydrogène, un radical alcoyle, alcoxyalcoyle, cycloalcoyle le cas échéant substitué (où le cas échéant, un radical méthylène est remplacé par l'atome d'oxygène ou de soufre) ou un radical phényle, hétaryle, phénylalcoyle ou hétarylalcoyle, chaque fois le cas échéant substitué,
Q², Q³, Q⁴ représentent indépendamment l'un de l'autre, l'atome d'hydrogène ou un radical alcoyle,
Q¹ et Q² avec l'atome de carbone sur lequel ils sont liés, représentent un cycle saturé ou insaturé, contenant le cas échéant un hétéroatome, non substitué ou substitué.

2. Composés de la formule (I) selon la revendication 1, dans laquelle :
W représente un atome d'halogène, un radical alcoyle en C₁-C₆, alcényle en C₂-C₆, alcynyle en C₂-C₆, alcoxy en C₁-C₆, halogénoalcoyle en C₁-C₄ ou halogénoalcoxy en C₁-C₄ ou cyano,
X représente l'atome d'hydrogène, d'halogène, un radical alcoyle en C₁-C₆, alcoxy en C₁-C₆, halogénoalcoyle en C₁-C₄, halogénoalcoxy en C₁-C₄ ou cyano,
Y représente l'atome d'hydrogène, d'halogène, un radical alcoyle en C₁-C₆, alcoxy en C₁-C₆, halogénoalcoyle en C₁-C₄, halogénoalcoxy en C₁-C₄, cyano ou le radical : 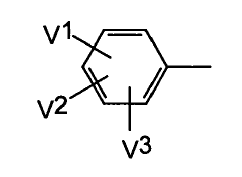
V¹ représente l'atome d'hydrogène, d'halogène, un radical alcoyle en C₁-C₁₂, alcoxy en C₁-C₆, alcoylthio en C₁-C₆, alcoylsulfinyle en C₁-C₆, alcoylsulfonyle en C₁-C₆, halogénoalcoyle en C₁-C₄, halogénoalcoxy en C₁-C₄, nitro, cyano ou un radical phényle, phénoxy, phénoxy-alcoyle en C₁-C₄, phényl-alcoxy en C₁-C₄, phénylthio-alcoyle en C₁-C₄ ou phényl-alcoylthio en C₁-C₄, chaque fois, le cas échéant une ou plusieurs fois substitué par un atome d'halogène, par un radical alcoyle en C₁-C₆, alcoxy en C₁-C₆, halogénoalcoyle en C₁-C₄, halogénoalcoxy en C₁-C₄, nitro ou cyano,
V² et V³ représentent indépendamment l'un de l'autre, l'atome d'hydrogène, d'halogène, un radical alcoyle en C₁-C₆, alcoxy en C₁-C₆, halogénoalcoyle en C₁-C₄ ou halogénoalcoxy en C₁-C₄,
V¹ et V² avec l'atome de carbone, sur lequel ils sont liés, représentent un cycle de 5 ou 6 membres, le cas échéant substitué par un ou plusieurs radicaux alcoyle en C₁-C₄ ou un ou plusieurs atomes d'halogène, dans lequel le cas échéant, un à deux atomes de carbone peuvent être remplacés par un atome d'oxygène, de soufre ou d'azote,
Z représente l'atome d'hydrogène, d'halogène, un radical alcoyle en C₁-C₆, alcoxy en C₁-C₆, halogénoalcoyle en C₁-C₄, halogénoalcoxy en C₁-C₄ ou cyano,
G représente un atome d'halogène ou le radical nitro,
m représente le nombre 0 ou 1,
A représente l'atome d'hydrogène, un radical alcoyle en C₁-C₁₂, alcényle en C₃-C₈, alcoxy en C₁-C₆-alcoyle en C₁-C₄, chaque fois le cas échéant substitué par un ou plusieurs atomes d'halogène, un radical cycloalcoyle en C₃-C₈ ou cycloalcoyl en C₃-C₈-alcoyle en C₁-C₄, chaque fois le cas échéant substitué par un ou plusieurs atomes d'halogène, un ou plusieurs radicaux alcoyle en C₁-C₄ ou alcoxy en C₁-C₄, dans lequel le cas échéant un ou deux atomes cycliques non directement voisins sont remplacés par un atome d'oxygène et/ou de soufre, ou un radical phényle, benzyle, hétaryle avec 5 ou 6 atomes cycliques ou hétaryl-alcoyle en C₁-C₄ avec 5 à 6 atomes cycliques, chaque fois le cas échéant substitué par un ou plusieurs atomes d'halogène, un ou plusieurs radicaux alcoyle en C₁-C₆, halogénoalcoyle en C₁-C₆, alcoxy en C₁-C₆, halogénoalcoxy en C₁-C₆, cyano ou nitro,
B représente l'atome d'hydrogène ou un radical alcoyle en C₁-C₆,
A, B et l'atome de carbone sur lequel ils sont liés, représentent un radical cycloalcoyle en C₃-C₁₀ saturé ou cycloalcoyle en C₃-C₁₀ insaturé, où le cas échéant, un atome cyclique est remplacé par un atome d'oxygène ou de soufre et qui est substitué le cas échéant, une ou deux fois, par un radical alcoyle en C₁-C₆, cycloalcoyle en C₃-C₈, halogénoalcoyle en C₁-C₆, alcoxy en C₁-C₆, alcoylthio en C₁-C₆, halogène ou phényle,
A et Q¹ ensemble, représentent un radical alcanediyle en C₃-C₆, le cas échéant substitué une ou deux fois, de manière identique ou différente, par un radical alcoyle en C₁-C₄ ou alcoxy en C₁-C₄,
Q¹ représente l'atome d'hydrogène, un radical alcoyle en C₁-C₆, alcoxy en C₁-C₆-alcoyle en C₁-C₂, un radical cycloalcoyle en C₃-C₈, le cas échéant substitué par un ou plusieurs atomes de fluor, de chlore, un ou plusieurs radicaux alcoyle en C₁-C₄, halogénoalcoyle en C₁-C₂ ou alcoxy en C₁-C₄, où le cas échéant, un radical méthylène est remplacé par l'atome d'oxygène ou de soufre, ou un radical phényle, pyridyle, thiényle, thiazolyle, phényl-alcoyle en C₁-C₄, pyridyl-alcoyle en C₁-C₂ ou thiazolyl-alcoyle en C₁-C₂, chaque fois le cas échéant substitué par un ou plusieurs atomes d'halogène, un ou plusieurs radicaux alcoyle en C₁-C₄, alcoxy en C₁-C₄, halogénoalcoyle en C₁-C₂, halogénoalcoxy en C₁-C₂, cyano ou nitro,
Q²p Q³, Q⁴ représentent indépendamment l'un de l'autre, l'atome d'hydrogène ou un radical alcoyle en C₁-C₄,
Q¹ et Q² avec l'atome de carbone sur lequel ils sont liés, représentent un cycloalcoyle en C₃-C₇, le cas échéant substitué par un ou plusieurs radicaux alcoyle en C₁-C₆, alcoxy en C₁-C₆ ou halogénoalcoyle en C₁-C₂, dans lequel un atome cyclique est le cas échéant remplacé par un atome d'oxygène ou de soufre.

3. Composés de la formule (I) selon la revendication 1, dans laquelle :
W représente l'atome de fluor, de chlore, de brome, un radical alcoyle en C₁-C₄, alcoxy en C₁-C₄, halogénoalcoyle en C₁-C₂, halogénoalcoxy en C₁-C₂ ou cyano,
X représente l'atome d'hydrogène, de fluor, de chlore, de brome, un radical alcoyle en C₁-C₄ ou alcoxy en C₁-C₄, halogénoalcoyle en C₁-C₄, halogénoalcoxy en C₁-C₄ ou cyano,
Y représente l'atome d'hydrogène, de fluor, de chlore, de brome, un radical alcoyle en C₁-C₆, alcoxy en C₁-C₆, halogénoalcoyle en C₁-C₂, halogénoalcoxy en C₁-C₂, cyano ou le radical: 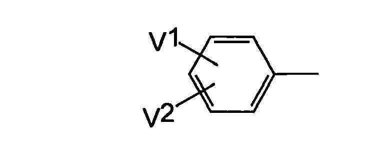
V¹ représente l'atome d'hydrogène, de fluor, de chlore, de brome, un radical alcoyle en C₁-C₆, alcoxy en C₁-C₄, halogénoalcoyle en C₁-C₂, halogénoalcoxy en C₁-C₂, nitro, cyano ou un radical phényle ou phénoxy, chaque fois, le cas échéant une ou plusieurs fois substitué par l'atome de fluor, de chlore, de brome, un radical alcoyle en C₁-C₄, alcoxy en C₁-C₄, halogénoalcoyle en C₁-C₂, halogénoalcoxy en C₁-C₂, nitro ou cyano,
V² représente l'atome d'hydrogène, de fluor, de chlore, un radical alcoyle en C₁-C₄, alcoxy en C₁-C₄, halogénoalcoyle en C₁-C₂ ou halogénoalcoxy en C₁-C₂,
V¹ et V² avec l'atome de carbone, sur lequel ils sont liés, représentent un cycle de 5 ou 6 membres, le cas échéant substitué par un ou plusieurs atomes de fluor ou un ou plusieurs radicaux méthyle, dans lequel cycle le cas échéant, un à deux atomes de carbone peuvent être remplacés par un atome d'oxygène,
Z représente l'atome d'hydrogène, de fluor, de chlore, de brome, un radical alcoyle en C₁-C₄, alcoxy en C₁-C₄, halogénoalcoyle en C₁-C₂, halogénoalcoxy en C₁-C₂ ou cyano,
G représente l'atome de chlore, de brome, ou le radical nitro,
m représente le nombre 0 ou 1,
A représente l'atome d'hydrogène, un radical alcoyle en C₁-C₈ ou alcoxy en C₁-C₄-alcoyle en C₁-C₂, chaque fois le cas échéant substitué par un ou plusieurs atomes de fluor, un radical cycloalcoyle en C₅-C₆ ou cycloalcoyl en C₃-C₆-alcoyle en C₁-C₂, chaque fois le cas échéant substitué par un ou plusieurs atomes de fluor, un ou plusieurs radicaux méthyle, éthyle ou méthoxy et dans lequel le cas échéant un atome cyclique est remplacé par un atome d'oxygène et/ou de soufre, ou un radical phényle ou benzyle, chaque fois le cas échéant substitué par un ou plusieurs atomes de fluor, de chlore, de brome, un ou plusieurs radicaux alcoyle en C₁-C₄, halogénoalcoyle en C₁-C₂, alcoxy en C₁-C₄, halogénoalcoxy en C₁-C₂,
B représente l'atome d'hydrogène ou un radical alcoyle en C₁-C₄,
A, B et l'atome de carbone sur lequel ils sont liés, représentent un radical cycloalcoyle en C₅-C₇ saturé où le cas échéant, un atome cyclique est remplacé par un atome d'oxygène et qui est substitué le cas échéant, une fois, par un radical alcoyle en C₁-C₄, trifluorométhyle ou alcoxy en C₁-C₄,
avec la condition qu'alors, Q¹ représente seulement l'atome d'hydrogène,
A et Q¹ ensemble, représentent un radical alcanediyle en C₃-C₄, le cas échéant substitué une ou deux fois, par le radical méthyle, éthyle, méthoxy ou éthoxy,
Q¹ représente l'atome d'hydrogène, un radical alcoyle en C₁-C₆, alcoxy en C₁-C₄-alcoyle en C₁-C₂, ou cycloalcoyle en C₃-C₆, le cas échéant substitué par un ou plusieurs radicaux méthyle ou méthoxy, où le cas échéant, un radical méthylène est remplacé par l'atome d'oxygène, ou un radical phényle ou benzyle, chaque fois le cas échéant substitué par un ou plusieurs atomes de fluor, de chlore, de brome, un ou plusieurs radicaux alcoyle en C₁-C₄, alcoxy en C₁-C₄, trifluorométhyle ou trifluorométhoxy,
Q², Q³, Q⁴ représentent indépendamment l'un de l'autre, l'atome d'hydrogène, le radical méthyle ou éthyle
Q¹ et Q² avec l'atome de carbone sur lequel ils sont liés, représentent un cycloalcoyle en C₅-C₆ saturé, le cas échéant substitué par un ou plusieurs radicaux alcoyle en C₁-C₄ ou alcoxy en C₁-C₄, dans lequel un atome cyclique est le cas échéant remplacé par un atome d'oxygène,
avec la condition qu'alors, A représente seulement l'atome d'hydrogène.

4. Composés de la formule (I) selon la revendication 1, dans laquelle :
W représente l'atome de chlore, de brome, le radical méthyle, éthyle, propyle, méthoxy, éthoxy, trifluorométhyle, difluorométhoxy, trifluorométhoxy ou cyano,
X représente l'atome d'hydrogène, de chlore, de brome, le radical méthyle, éthyle, propyle, méthoxy ou éthoxy,
Y représente l'atome d'hydrogène, de fluor, de chlore, de brome, le radical méthyle, éthyle, n-propyle, i-butyle, CH(CH₃)-i-butyle, méthoxy, éthoxy, trifluorométhyle, trifluorométhoxy, difluorométhoxy, cyano, ou le radical : 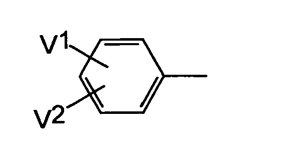
V¹ représente l'atome d'hydrogène, de fluor, de chlore, de brome, le radical méthyle, éthyle, n-propyle, isopropyle, n-butyle, i-butyle, t-butyle, méthoxy, éthoxy, n-propoxy, isopropoxy, trifluorométhyle ou trifluorométhoxy,
V² représente l'atome d'hydrogène, de fluor, de chlore, le radical méthyle, éthyle, n-propyle, isopropyle, méthoxy, éthoxy ou trifluorométhyle,
Z représente l'atome d'hydrogène, de fluor, de chlore, de brome, le radical méthyle, éthyle, propyle, méthoxy, éthoxy, trifluorométhyle, trifluorométhoxy, difluorométhoxy ou cyano,
G représente l'atome de chlore, de brome ou le radical nitro,
m représente le nombre 0 ou 1,
A représente l'atome d'hydrogène, le radical méthyle, éthyle, n-propyle, isopropyle, n-butyle, isobutyle, méthoxyméthyle ou éthoxyméthyle,
B représente l'atome d'hydrogène le radical méthyle ou éthyle,
A, B et l'atome de carbone sur lequel ils sont liés, représentent un radical cycloalcoyle en C₅-C₇ saturé, où le cas échéant, un atome cyclique est remplacé par un atome d'oxygène et qui est substitué le cas échéant, une fois, par le radical méthyle, éthyle, isopropyle, trifluorométhyle, méthoxy, éthoxy, n-propoxy, n-butoxy ou isobutoxy,
avec la condition qu'alors, Q¹, Q², Q³ et Q⁴ représentent seulement l'atome d'hydrogène,
A et Q¹ ensemble, représentent un radical alcandiyle en C₃-C₄,
Q¹ représente l'atome d'hydrogène, le radical méthyle, éthyle, n-propyle, isopropyle, n-hexyle, cyclopropyle, cyclopentyle ou cyclohexyle,
Q², Q³, Q⁴ représentent indépendamment l'un de l'autre, l'atome d'hydrogène, le radical méthyle ou éthyle,
Q¹ et Q² avec l'atome de carbone sur lequel ils sont liés, représentent un cycloalcoyle en C₅-C₆ saturé, le cas échéant substitué par un ou plusieurs radicaux méthyle, éthyle, méthoxy, éthoxy, n-propoxy ou n-butoxy, dans lequel un atome cyclique est le cas échéant remplacé par un atome d'oxygène,
avec la condition qu'alors, A, B, Q³ et Q⁴ représentent seulement l'atome d'hydrogène.

5. Composés de la formule (I) selon la revendication 1, dans laquelle :
W représente le radical méthyle ou l'atome de chlore,
Y représente le radical phényle le cas échéant substitué une ou deux fois, par l'atome de chlore ou de fluor, ou représente l'atome de chlore, de brome, le radical méthyle, éthyle, n-propyle, i-butyle, CH(CH₃)-i-butyle ou trifluorométhyle,
Z représente l'atome d'hydrogène,
G représente l'atome de chlore,
m représente le nombre 0 ou 1,
A représente l'atome d'hydrogène ou le radical méthyle,
B représente l'atome d'hydrogène ou le radical méthyle,
A, B et l'atome de carbone sur lequel ils sont liés, représentent un radical cycloalcoyle en C₅-C₇, avec la condition qu'alors, Q¹ et Q² représentent seulement l'atome d'hydrogène,
Q¹ représente l'atome d'hydrogène, le radical méthyle, éthyle, n-propyle ou n-hexyle,
Q² représente l'atome d'hydrogène ou le radical méthyle,
Q¹ et Q² avec l'atome de carbone sur lequel ils sont liés, représentent un radical cycloalcoyle en C₅-C₆, avec la condition qu'alors, A et B représentent seulement l'atome d'hydrogène ;
Q³ représente l'atome d'hydrogène,
Q⁴ représente l'atome d'hydrogène.

6. Procédé de préparation de composés de la formule (I) selon la revendication 1, **caractérisé en ce que**,
A) pour l'obtention de composés des formules (I-1) à (1-2) : 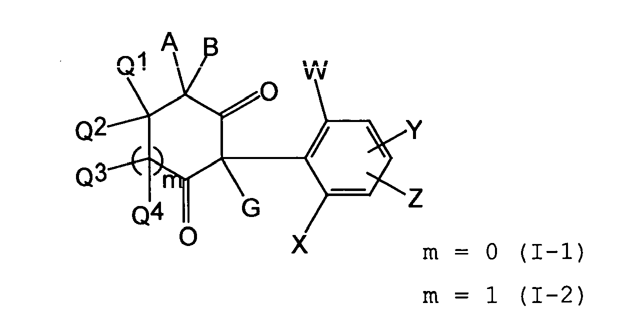 dans laquelle :
A, B, Q¹, Q², Q³, Q⁴, W, X, Y et Z ont la signification donnée ci-dessus, et
G représente un atome d'halogène,
on fait réagir des composés des formules (II-1) à (II-2): 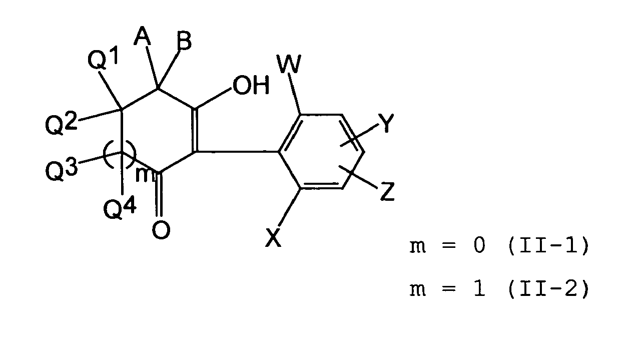
dans laquelle:
A, B, Q¹, Q², Q³, Q⁴, W, X, Y et Z ont la signification donnée ci-dessus,
avec un agent d'halogénation en présence d'un solvant et le cas échéant, en présence d'un initiateur de radicaux, et
B) pour l'obtention de composés des formules (I-1) à (1-2) : 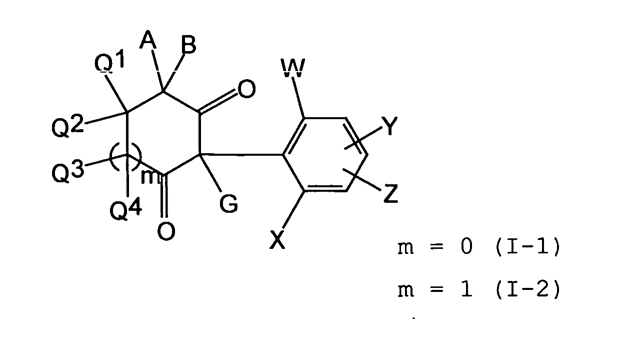 dans laquelle:
A, B, Q¹, Q², Q³, Q⁴, W, X, Y et Z ont la signification donnée ci-dessus, et
G représente un radical nitro,
on fait réagir des composés des formules (II-1) à (II-2) : 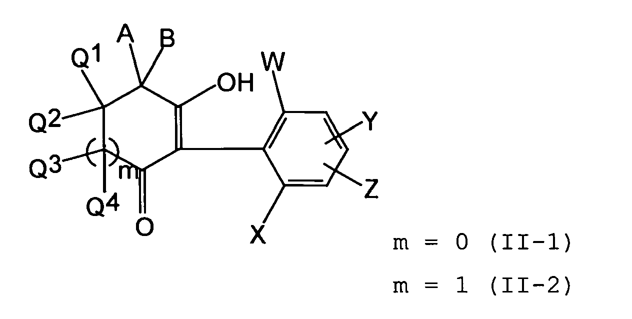
dans laquelle :
A, B, Q¹ Q², Q³, Q⁴ W, X, Y et Z ont la signification donnée ci-dessus,
avec un agent de nitration, comme par exemple l'acide nitrique, en présence d'un solvant.

7. Agent pour lutter contre des parasites, des végétaux non désirés et/ou des microorganismes non désirés, **caractérisé par** une teneur en au mois un composé de la formule (I) selon la revendication 1.

8. Procédé pour lutter contre des parasites animaux, des végétaux non désirés et/ou des microorganismes non désirés, **caractérisé en ce que** l'on fait agir des composés de la formule (I) selon la revendication 1, sur les parasites, végétaux non désirés, microorganismes non désirés et/ou leur biotope.

9. Utilisation des composés de la formule (I) selon la revendication 1, pour lutter contre des parasites animaux, des végétaux non désirés et/ou des microorganismes non désirés.

10. Procédé de préparation d'agents pour lutter contre des parasites, des végétaux non désirés et/ou des microorganismes non désirés, **caractérisé en ce que** l'on mélange des composés de la formule (1) selon la revendication 1, avec des diluants et/ou des agents tensioactifs.

11. Utilisation de composés de la formule (1) selon la revendication 1, pour la préparation d'agents pour lutter contre des parasites, des végétaux non désirés et/ou des microorganismes non désirés.
